# EUROPEAN PATENT SPECIFICATION

(11) **EP 3 023 494 B1**
(45) Date of publication and mention of the grant of the patent: **28.11.2018**
(21) Application number: 15199731.9
(22) Date of filing: 11.06.2009
(51) Int. Cl.: C12N 15/10, C12N 15/11, C07H 21/00, C12Q 1/68, C40B 50/06, C40B 40/06

(54) **METHOD OF SYNTHESIZING POLYNUCLEOTIDE VARIANTS**
VERFAHREN ZUR SYNTHESE VON POLYNUKLEOTIDVARIANTEN
PROCÉDÉ DE SYNTHÈSE DE VARIANTS POLYNUCLÉOTIDIQUES

(30) Priority: 13.06.2008 US 61581 P
(43) Date of publication of application: 25.05.2016
(62) Divisional of application: 09763625.2
(73) Proprietor: Codexis, Inc., Redwood City, CA 94063 (US)
(72) Inventor: COLBECK, Jeffrey, 5 Menlo Park, CA 94025 (US); MIJTS, Benjamin, Redwood City, CA 94063 (US); GIVER, Lorraine, Jean, Sunnyvale, CA 94085 (US); FOX, Richard, J., Kirkwood, MO 63122 (US)
(74) Representative: Chapman, Desmond Mark

(56) References cited:
- JP-A- 2003 265 187
- US-A1- 2002 160 380
- YINGFENG AN ET AL: "A rapid and efficient method for multiple-site mutagenesis with a modified overlap extension PCR", 1 October 2005 (2005-10-01), APPLIED MICROBIOLOGY AND BIOTECHNOLOGY, SPRINGER, BERLIN, DE, PAGE(S) 774 - 778, XP019331974, ISSN: 1432-0614 * page 774, right-hand column, last paragraph - page 776, left-hand column, paragraph 1; figure 2 *
- HO S N ET AL: "SITE-DIRECTED MUTAGENESIS BY OVERLAP EXTENSION USING THE POLYMERASE CHAIN REACTION", GENE, ELSEVIER, AMSTERDAM, NL, vol. 77, no. 1, 1 January 1989 (1989-01-01), pages 51-59, XP000272761, ISSN: 0378-1119, DOI: 10.1016/0378-1119(89)90358-2
- URBAN A ET AL: "A RAPID AND EFFICIENT METHOD FOR SITE-DIRECTED MUTAGENESIS USING ONE-STEP OVERLAP EXTENSION PCR", 1 June 1997 (1997-06-01), NUCLEIC ACIDS RESEARCH, OXFORD UNIVERSITY PRESS, SURREY, GB, PAGE(S) 2227/2228, XP002043113, ISSN: 0305-1048 * the whole document *
- WATARU ITO ET AL: "A GENERAL METHOD FOR INTRODUCING A SERIES OF MUTATIONS INTO CLONED DNA USING THE POLYMERASE CHAIN REACTION", 1 January 1991 (1991-01-01), GENE, ELSEVIER, AMSTERDAM, NL, PAGE(S) 67 - 70, XP000303855, ISSN: 0378-1119 * page 68, left-hand column, paragraph 2 - page 69, right-hand column, paragraph 1; figure 1 *
- JENSEN PIA HONNERUP ET AL: "Combination primer polymerase chain reaction for multi-site mutagenesis of close proximity sites.", December 2005 (2005-12), JOURNAL OF BIOMOLECULAR TECHNIQUES : JBT DEC 2005 LNKD- PUBMED:16522854, VOL. 16, NR. 4, PAGE(S) 336 - 340, XP002666007, ISSN: 1524-0215 * page 337, left-hand column, paragraph 2 - page 339, left-hand column, paragraph 1; figures 1-4 *
- HUGHES ET AL: "Cost-Effective High-Throughput Fully Automated Construction of a Multiplex Library of Mutagenized Open Reading Frames for an Insecticidal Peptide Using a Plasmid-Based Functional Proteomic Robotic Workcell with Improved Vacuum System", JOURNAL OF THE ASSOCIATION FOR LABORATORY AUTOMATION, ELSEVIER, vol. 12, no. 4, 14 July 2007 (2007-07-14), pages 202-212, XP022163242, ISSN: 1535-5535, DOI: 10.1016/J.JALA.2007.04.007
- HIBBERT E G ET AL: "Directed evolution of transketolase substrate specificity towards an aliphatic aldehyde", JOURNAL OF BIOTECHNOLOGY, ELSEVIER, AMSTERDAM, NL, vol. 134, no. 3-4, 30 April 2008 (2008-04-30), pages 240-245, XP022587997, ISSN: 0168-1656, DOI: 10.1016/J.JBIOTEC.2008.01.018 [retrieved on 2008-02-13]
- HIBBERT ET AL: "Directed evolution of transketolase activity on non-phosphorylated substrates", JOURNAL OF BIOTECHNOLOGY, ELSEVIER, AMSTERDAM, NL, vol. 131, no. 4, 20 September 2007 (2007-09-20), pages 425-432, XP022259320, ISSN: 0168-1656, DOI: 10.1016/J.JBIOTEC.2007.07.949

## Description

### 1. BACKGROUND

Various techniques of *in silico* and *in vitro* based directed evolution of protein function have allowed the generation of proteins with novel properties. For example, cytochrome P450 enzymes have been evolved to have activity against substrates not normally recognized by the naturally occurring enzyme (see, e.g., Landwehr et al., 2007, Chem Biol 14(3):269-78; Kubo et al., 2006, Chemistry 12(4):1216-20.). Typically, for generating such new enzymes, a polynucleotide encoding a reference polypeptide, such as a wild type enzyme, is subjected to mutagenesis to generate polynucleotides encoding polypeptide variants with changes in amino acid sequence. Screening of the variants for a desired property, such as an improvement in an enzyme stability or activity against new substrates, allows the identification of the amino acid residues associated with the changed property. However, not all combinations of the mutations will be present in the population of screened variants. For example, a mutation associated with thermal stability of an enzyme may not be found in association with a mutation associated with a change in substrate specificity. This bias in the population can arise from various factors, including, among others, the parental amino acid sequence encoded by the polynucleotide used for mutagenesis, possible selection against the combination during *in vivo* propagation of the polynucleotide, and the bias in the technique used for mutagenesis (e.g., use of polymerases to introduce errors). Yingfeng et al. (Appl Microbiol Biotechnol (2005) 68: 774-778) describe a method of performing site directed mutagenesis based on overlap extension by polymerase chain reaction (OE-PCR). JP 2003 265187 describes construction of a comprehensive mutant gene library where at least one base is mutated in order to analyze p53 gene.

Because the mutations at defined amino acid residue positions of a reference polypeptide sequence can provide a wealth of information about the polypeptide's biological activities, once mutations have been initially identified, it is desirable to prepare various combinations of the mutations not found in the initial set of screened variants that can be tested for the desired property. *In silico* based selection of defined mutations or sets of mutations provide a framework for generating a large number of possible mutation combinations. For example, mutations affecting substrate specificity can be combined with mutations affecting other enzyme properties, including, among others, enzyme activity, thermal stability, and inhibitor resistance. Typically, the approach to generating these polypeptides having novel combinations of mutations is to synthesize individual species (*i.e.,* synthesis of each polynucleotide encoding the mutant gene). This can be accomplished by chemical and/or enzymatic synthesis of the polynucleotide in combination with standard recombination techniques. Such *de novo* synthesis techniques require whole gene synthesis of each polynucleotide variant and/or synthesis of large numbers of oligonucleotide primers which are then used to synthesize whole polynucleotide variant (e.g., via ION-PCR). These techniques require more oligonucleotide syntheses and result in lower yields of variants having the correct sequence. Consequently, if the data set of mutations is large, the cost and efficiency of generating the mutation combinations can limit the ability to screen a large number of novel combinations. Thus, efficient and cost effective methods of generating polynucleotides encoding combinations of defined mutations are desirable.

### 2. SUMMARY

The invention provides a method of synthesizing a plurality of polynucleotide variants each having at least one defined nucleotide difference relative to a reference polynucleotide sequence, the method comprising:
(a) separately amplifying a reference polynucleotide template with each of a plurality of pairs of forward and reverse primers, wherein the plurality of pairs of forward and reverse primers comprises a plurality of defined nucleotide differences and wherein each pair generates an amplicon comprising a sequence capable of binding to an adjacent overlapping sequence of at least one other amplicon, thereby generating an addressable library of amplicons each corresponding to a segment of the reference polynucleotide sequence having a defined nucleotide difference;
(b) separately assembling a plurality of sets of amplicons from the addressable library of amplicons, wherein each set comprises amplicons having adjacent overlapping sequences capable of binding to form the full length of the reference polynucleotide sequence;
(c) replicating the plurality of sets of assembled amplicons, thereby synthesizing a plurality of polynucleotide variants each having at least one defined nucleotide difference relative to the reference polynucleotide,
wherein the reference polynucleotide encodes a reference polypeptide and each of the plurality of polynucleotide variants encodes a polypeptide having at least one defined amino acid sequence difference as compared to the reference polypeptide,
and wherein the method is used to synthesize a polynucleotide variant encoding a polypeptide with an improvement in a desired property of a reference polypeptide encoded by the reference polynucleotide.

The invention also provides an addressable library of polynucleotide variants comprising a plurality of polynucleotide variants synthesized according to the above method, each having at least one defined nucleotide difference relative to a reference polynucleotide sequence, wherein the library comprises at least 10 polynucleotide variants, wherein at least one of the polynucleotide variants comprises at least two defined nucleotide differences relative to the reference polynucleotide sequence, wherein the reference polynucleotide encodes a reference polypeptide and each of the plurality of polynucleotide variants encodes a polypeptide having at least one defined amino acid sequence difference as compared to the reference polypeptide, and wherein the library comprises a polynucleotide variant encoding a polypeptide with an improvement in a desired property of a reference polypeptide encoded by the reference polynucleotide.

The invention also provides an addressable library of amplicons, wherein each member of the library of amplicons comprises at least one defined nucleotide difference relative to a reference polynucleotide sequence and an overlapping adjacent region capable of binding to the overlapping adjacent region of at least one other amplicon in the library, wherein the library comprises at least 5 different amplicons, wherein the library of amplicons comprises members for assembling two or more different polynucleotide variants comprising defined nucleotide differences relative to the reference polynucleotide sequence, wherein the reference polynucleotide encodes a reference polypeptide and each of the plurality of polynucleotide variants encodes a polypeptide having at least one defined amino acid sequence difference as compared to the reference polypeptide, and wherein the library of amplicons comprises at least one set of amplicons capable of binding to form the full length of the reference polynucleotide sequence and encoding a polypeptide with an improvement in a desired property of a reference polypeptide encoded by the reference polynucleotide.

The invention also provides an addressable library of clones, each clone capable of generating a different variant polypeptide having at least one defined amino acid sequence difference as compared to a reference polypeptide, generated by cloning the addressable library of polynucleotide variants into an expression system.

The invention also provides an addressable library of transformed cells, each cell capable of generating a different variant polypeptide having at least one defined amino acid sequence difference as compared to the reference polypeptide, generated by transforming the addressable library of clones into cells.

The invention also provides a robotic system comprising the addressable library of polynucleotide variants, the addressable library of amplicons according and/or the addressable library of transformed cells.

The present disclosure relates to methods of efficiently generating polynucleotides having different combinations of defined sequence changes (*e.g.,* desired amino acid mutations) as compared to a reference polynucleotide sequence. The methods are based on use of a library of polynucleotide fragments (*i.e.,* amplicons) that have overlapping adjacent regions such that a sets of the polynucleotide fragments can be assembled to generate a plurality of polynucleotide variants each having a defined set of sequence changes. Selected forward and reverse primers are used to introduce the sequence changes by amplifying a reference polynucleotide template and thereby generate polynucleotide fragments comprising the defined sequence changes. The library is designed to have sufficient polynucleotide fragments to assemble at least two different polynucleotide variant sequences. In some embodiments, the library of polynucleotide fragments contain members that have all of the defined differences in polynucleotide sequence (*e.g.,* desired nucleotide changes) as compared to a reference sequence such that all sequence permutations can be assembled. In some embodiments, the polynucleotides can be designed to encode polypeptides having defined differences in amino acid sequence as compared to a reference amino acid sequence.

The methods of the present disclosure are capable of producing large libraries of polynucleotide variant sequences having defined nucleotide differences (e.g., libraries of 10, 50, 100, 150, or more variants, each having 1, 2, 3, 5, 9, 12, 15, or more, desired changes), with relatively few (e.g., compared to whole gene synthesis methods), and relatively short (e.g., 35-mer or less) oligonucleotides, and wherein the average percentage of correct sequences is surprisingly high (e.g., at least 65%, 75%, 85%, 95%, or more).

In some embodiments, the method of forming polynucleotides encoding polypeptide variants can comprise: selecting a plurality of defined amino acid residue differences relative to a reference amino acid sequence; defining overlapping segments of a polynucleotide sequence encoding the polypeptide with the different amino acid sequence or the reference amino acid sequence, each segment being bounded by a set of forward and reverse primer binding sequences and wherein a polynucleotide sequence difference encoding each of the plurality of amino acid residue differences is encompassed in the primer binding sequence; amplifying each segment with the set of forward and reverse primers, wherein selected forward and/or reverse primers contain the polynucleotide sequence differences, to generate a library of amplicons comprising members encoding the amino acid residue differences, wherein the library comprises members for assembling two or more different amino acid sequence permutations of the defined amino acid differences; assembling from the library a set of amplicons having complementary overlapping adjacent regions, wherein the set of amplicons together encode the polypeptide with a defined amino acid sequence permutation having one or more amino acid residue differences; and replicating the set of assembled amplicons to synthesize the polynucleotide encoding the polypeptide.

Further described herein are methods of generating the polynucleotide fragment library, where the method comprises: (a) generating a plurality of permutations of amino acid sequences differing from a reference amino acid sequence based on a plurality of defined amino acid residue differences from the reference amino acid sequence, and for each permutation; (i) determining a polynucleotide sequence encoding the amino acid sequence permutation based on a reference polynucleotide sequence; (ii) identifying a change in polynucleotide sequence encoding an amino acid residue difference as compared to a reference amino acid sequence, and determining the proximity of a nearest-neighbor change in polynucleotide sequence encoding another amino acid residue difference in the amino acid sequence permutation; (iii) selecting a forward oligonucleotide primer having a sequence encoding the amino acid residue difference, and optionally including the nearest-neighbor change in polynucleotide sequence in the same forward oligonucleotide primer if proximate to the first change in polynucleotide sequence; (iv) identifying the next change in polynucleotide sequence or until the end of the polynucleotide is reached, and selecting a reverse oligonucleotide primer for amplifying a polynucleotide fragment with the forward oligonucleotide primers, wherein the reverse primer optionally encodes the next change in amino acid residue difference; (v) reiterating steps (ii) to (iv) for each change in polynucleotide sequence encoding an amino acid residue difference such that all changes in the polynucleotide sequence are present on oligonucleotide primers; and (b) amplifying with each set of forward and reverse oligonucleotide primers to generate the library of overlapping amplicons having members encoding amino acid residue differences.

In another aspect, the present disclosure provides library of such polynucleotide fragments (i.e., amplicons) for assembling the polynucleotides. In some embodiments, the plurality of polynucleotide fragments comprises polynucleotide fragments with overlapping adjacent regions, each polynucleotide fragment being bounded by primer binding sequences for forward and reverse primers, wherein the plurality of polynucleotide fragments have members that encode in the primer binding sequences a specific amino acid residue difference from a defined plurality of amino acid residue differences relative to a reference amino acid sequence such that the plurality of polynucleotide fragments encode all of a selected plurality of amino acid residues differences from the defined plurality of amino acid residue differences; and wherein the plurality of polynucleotide fragment comprises members for assembling two or more different amino acid sequence permutations of the defined amino acid differences. In some embodiments, the plurality of polynucleotide fragments comprises members sufficient for assembling all of the possible amino acid sequence permutations of the selected plurality of amino acid residue differences.

In another aspect, the present disclosure provides a method of synthesizing a plurality of polynucleotide variants each having a defined nucleotide difference relative to a reference polynucleotide sequence, wherein the method comprises: (a) separately amplifying a reference polynucleotide template with each of a plurality of pairs of forward and reverse primers, wherein the plurality of pairs of forward and reverse primers comprises the plurality of defined nucleotide differences and wherein each pair generates an amplicon comprising a sequence capable of binding to an adjacent overlapping sequence of at least one other amplicon; (b) separately assembling a plurality of sets of amplicons, wherein each set comprises amplicons having adjacent overlapping sequences capable of binding to form the full length of the reference polynucleotide sequence; and (c) replicating the plurality of sets of assembled amplicons, thereby synthesizing a plurality of polynucleotide variants.

In another embodiment, the disclosure provides a method of synthesizing a plurality of polynucleotide variants each having a defined nucleotide difference relative to a reference polynucleotide sequence, the method comprising: (a) selecting a plurality of defined nucleotide differences relative to the reference polynucleotide sequence; (b) defining a plurality of segments of the reference polynucleotide sequence, wherein each segment overlaps at least one adjacent segment and is bounded by a pair of forward and reverse primer binding sequences, wherein the forward and/or reverse primers comprise at least one of the plurality of defined nucleotide differences; (c) separately amplifying a reference polynucleotide template with each of a plurality of pairs of forward and reverse primers, wherein each pair of primers comprises at least one of the plurality of defined nucleotide differences, thereby generating an addressable library of amplicons each corresponding to a segment of the reference polynucleotide sequence having a defined nucleotide differences; (d) separately assembling a plurality of sets of amplicons from the addressable library of amplicons, wherein each set comprises amplicons corresponding to overlapping adjacent segments that comprise the full length of the reference polynucleotide sequence; and (e) replicating the plurality of sets of assembled amplicons, thereby synthesizing a plurality of polynucleotide variants.

In some embodiments of the methods of synthesizing a plurality of polynucleotide variants disclosed herein, the methods can be carried out wherein the reference polynucleotide encodes a reference polypeptide and each of the plurality of polynucleotide variants encodes a polypeptide having at least one amino acid sequence difference. In additional embodiments, the methods can be carried out wherein it further comprises the steps of: (i) cloning each of the plurality of polynucleotide variants into an expression vector; (ii) transforming cells with the expression vectors; (iii) screening the transformed cells for activity of the polypeptides encoded by the polynucleotide variants; or (iv) isolating at least one polypeptide encoded by the polynucleotide variants. Additionally, the methods can be carried out wherein each polynucleotide variant is assembled at a known position on an array.

In some embodiments of the methods of synthesizing a plurality of polynucleotide variants disclosed herein, the methods can be carried out wherein sequences of the plurality of forward and reverse primer sequences are generated by the steps of: (i) identifying a first defined difference in the polynucleotide variant sequence as compared to the reference sequence, and determining the proximity of a nearest-neighbor defined difference in the polynucleotide sequence; (ii) selecting a forward primer having a sequence comprising the first defined nucleotide difference, and optionally including any nearest-neighbor defined difference in the same forward primer if proximate to the first defined nucleotide difference; (iii) identifying a next defined difference in the polynucleotide variant sequence as compared to the reference sequence, and determining the proximity of a nearest-neighbor defined difference in the polynucleotide sequence, or identifying that the end of the polynucleotide variant has been reach; (iv) selecting a reverse primer having a sequence comprising the next defined nucleotide difference, and optionally including any nearest-neighbor defined difference in the same forward primer if proximate to the next defined nucleotide difference; and (v) repeating steps (iii) to (iv) for each defined difference in the polynucleotide variant sequence such that all defined difference are present on primers.

In some embodiments of the methods of synthesizing a plurality of polynucleotide variants disclosed herein, the plurality of polynucleotide variants comprises at least 10, 25, 35, 50, 75, 90, 120, 150, 180 or even more different polynucleotide variants.

In some embodiments of the methods of synthesizing a plurality of polynucleotide variants disclosed herein, at least one of the plurality of polynucleotide variants comprises at least 2, 3, 6, 9, 12, 15, 18, 21, 24, 27, 30, or even more defined nucleotide differences relative to the reference polynucleotide sequence. In some embodiments, two or more, or in some embodiments each of the plurality of polynucleotide variants comprises at least 1, 2, 3, 6, 9, 12, 15, 18, 21, 24, 27, 30, or even more defined nucleotide differences relative to the reference polynucleotide sequence.

In some embodiments of the methods of synthesizing a plurality of polynucleotide variants disclosed herein, at least one of the plurality of sets of amplicons comprises at least 3, at least 5, at least 7, at least 10, or more different amplicons. In some embodiments, two or more, or in some embodiments each of the plurality of sets of amplicons comprises at least 3, at least 5, at least 7, at least 10, or more different amplicons.

In some embodiments of the methods of synthesizing a plurality of polynucleotide variants disclosed herein, the length of the reference polynucleotide sequence is at least 500 bp, 750 bp, 1000 bp, 1250 bp, 1500 bp, or even longer.

In some embodiments of the methods of synthesizing a plurality of polynucleotide variants disclosed herein, the plurality of pairs of forward and reverse primers comprises 400 or fewer, 300 or fewer, 200 or fewer, 100 or fewer, 50 or fewer, or even 25 or fewer. In some embodiments, the plurality of pairs of forward and reverse primers comprises from 6 to about 200, from 6 to about 150, from 6 to about 100, from 6 to about 50, 6 to about 40, 6 to about 30, 6 to about 25, 6 to about 20, 6 to about 15, or even fewer different oligonucleotides, and wherein the lengths of the oligonucleotides are from about 20 to about 50 nucleotides, about 20 to about 40 nucleotides, or about 25 to about 35 nucleotides.

In some embodiments of the methods of synthesizing a plurality of polynucleotide variants disclosed herein, the average percentage of the synthesized plurality of polynucleotide variants comprising the correct sequence is at least about 65%, 75%, 85%, or 95%, or more.

In some embodiments, the disclosure provides methods of synthesizing a plurality of polynucleotide variants wherein any of the above described parameters (e.g., number of variants, number of defined nucleotide differences, length of the reference polynucleotide sequence, number of pairs of forward and reverse primers, length of primer oligonucleotides, and/or percentage of full length perfect sequences) are combined.

In addition to the above methods, the disclosure also provides an addressable library of polynucleotide variants comprising a plurality of polynucleotide variants or amplicons synthesized according to any of the above methods. Accordingly, in some embodiments the disclosure provides an addressable library of amplicons, wherein each member of the library of amplicons comprises at least one defined nucleotide difference relative to a reference polynucleotide sequence and an overlapping adjacent region capable of binding to the overlapping adjacent region of at least one other amplicon in the library, and wherein the plurality of amplicons comprise at least one set of amplicons capable of binding to form the full length of the reference polynucleotide sequence. In some embodiments, the addressable library of amplicons comprises members for assembling two or more different polynucleotide variants comprising defined nucleotide differences relative to the reference polynucleotide sequence. In some embodiments of the addressable library of amplicons, the reference polynucleotide sequence encodes a reference polypeptide and the plurality of amplicons comprises members sufficient for assembling all of the possible nucleotide differences encoding a selected plurality of amino acid residue differences.

Further disclosed herein are computer implemented methods for carrying out various steps of the methods described herein.

### 3. BRIEF DESCRIPTION OF THE FIGURES

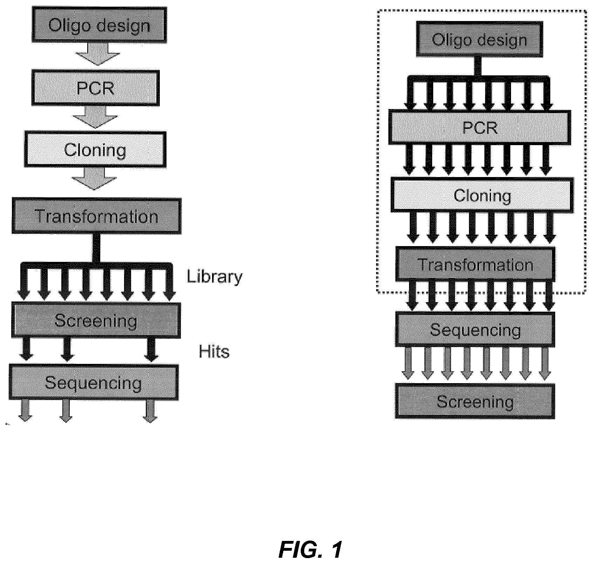

FIG. 1 shows a standard technique for generating polynucleotides encoding defined polypeptide variants (at left) as compared to the method described herein for using libraries of overlapping polynucleotide fragments (at right).
FIG. 2 provides a sample workflow scheme for generating libraries of overlapping polynucleotide fragments based on generating oligonucleotide primers for overlapping segments of a polynucleotide and use of the oligonucleotides in PCR reactions to generate libraries of overlapping polynucleotide fragments.
FIG. 3 shows the agarose gels of 96 samples (and 8 controls) resulting from assembling the overlapping polynucleotide fragments and replicating the assembled polynucleotide fragments to synthesize the polynucleotide variants encoding the desired polypeptide variant. Nearly every gel showed a single strong band indicating the expected length sequence was present.
FIG. 4 shows a flowchart for generating polynucleotides encoding variants by use of library of overlapping polynucleotide fragments.
FIG. 5 shows a flowchart for generating a library of oligonucleotide primers for generating a library of overlapping polynucleotide amplicons for each amino acid sequence permutation.
FIG. 6 shows a flowchart of instructions for automated creation and selection of oligonucleotide primers and overlapping oligonucleotide fragments.

### 4. DETAILED DESCRIPTION

As used in this specification and the appended claims, the singular forms "a", "an" and "the" include plural referents unless the context clearly indicates otherwise. Thus, for example, reference to "a protein" includes more than one protein, and reference to "a compound" refers to more than one compound.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of' or "consisting of."

The section headings used herein are for organizational purposes only and not to be construed as limiting the subject matter described. As used herein, the following terms are intended to have the following meanings.

### 4.1 Definitions

"Amplifying" and "amplification" as used herein incorporates its common usage and refers to the use of any suitable amplification methodology for generating or detecting any polynucleotide, recombinant or naturally expressed, amenable to amplification *in vivo* or *in vitro,* such as by polymerase chain reaction (PCR).

"Amplicon" refers to the product of the amplification reaction generated through the extension of either or both of a pair of amplification primers. An amplicon may contain exponentially amplified nucleic acids if both primers utilized hybridize to a target sequence. Alternatively, amplicons may be generated by linear amplification if one of the primers utilized does not hybridize to the target sequence. Thus, this term is used generically herein and does not imply the presence of exponentially amplified nucleic acids.

"Annealing" or "hybridization" refers to the base-pairing interactions of one nucleobase polymer with another that results in the formation of a double-stranded structure, a triplex structure or a quaternary structure. Annealing or hybridization can occur *via* Watson-Crick base-pairing interactions, but may be mediated by other hydrogen-bonding interactions, such as Hoogsteen base pairing.

"Assembling" refers to bringing together a plurality of polynucleotide fragments (e.g., amplicons) under conditions in which complementary regions between polynucleotides can anneal to form a hybridization complex, e.g., having a double stranded hybridized region with overhangs for the non-complementary regions. A plurality of polynucleotides can be assembled to form a larger polynucleotide that encodes a polypeptide of interest.

"Bridging polynucleotide" refers to a polynucleotide having complementary regions at the terminal regions such that one polynucleotide can anneal to one terminal region and another polynucleotide can anneal to the other terminal region of the bridging polynucleotide.

"Coding sequence" refers to that portion of a nucleic acid (*e.g.,* a gene) that encodes an amino acid sequence of a protein.

"Codon optimized" refers to changes in the codons of the polynucleotide encoding a protein to those preferentially used in a particular organism such that the encoded protein is efficiently expressed in the organism of interest. Although the genetic code is degenerate in that most amino acids are represented by several codons, called "synonyms" or "synonymous" codons, it is well known that codon usage by particular organisms is nonrandom and biased towards particular codon triplets. This codon usage bias may be higher in reference to a given gene, genes of common function or ancestral origin, highly expressed proteins versus low copy number proteins, and the aggregate protein coding regions of an organism's genome.

"Complementary" refers to the hybridization or base pairing between nucleotides or nucleic acids, such as, for instance, between the two strands of a double stranded DNA molecule or between an oligonucleotide primer and a primer binding site on a single stranded polynucleotide to be sequenced or amplified. Complementary nucleotides are, generally, A and T (or A and U), or C and G. Two single stranded RNA or DNA molecules are said to be substantially complementary when a polynucleotide (RNA or DNA) strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, e.g., M. Kanehisa, 1984, Nucleic Acids Res. 12:203. "Complementary to" is used herein to mean that the complementary sequence is substantially identical or identical to the reverse-complement of all or a portion of a reference polynucleotide sequence or that each nucleotide in one strand is able to form a base-pair with a nucleotide, or analog thereof in the opposite strand.

"Conservative amino acid substitutions" refer to the interchangeability of residues having similar side chains, and thus typically involves substitution of the amino acid in the polypeptide with amino acids within the same or similar defined class of amino acids. By way of example and not limitation, an amino acid with an aliphatic side chain may be substituted with another aliphatic amino acid, e.g., alanine, valine, leucine, isoleucine, and methionine; an amino acid with hydroxyl side chain is substituted with another amino acid with a hydroxyl side chain, e.g., serine and threonine; an amino acids having aromatic side chains is substituted with another amino acid having an aromatic side chain, e.g., phenylalanine, tyrosine, tryptophan, and histidine; an amino acid with a basic side chain is substituted with another amino acid with a basis side chain, e.g., lysine, arginine, and histidine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Control sequence" refers to a polynucleotide sequence used to effect the expression of coding and non-coding sequences to which they are associated. The nature of such control sequences differs depending upon the host organism. Control sequences generally include promoter, ribosomal binding site, and transcription termination sequence. The term
"control sequences" is intended to include components whose presence can influence expression, and can also include additional components whose presence is advantageous, for example, leader sequences and fusion partner sequences.

"Defined difference" as used herein in the context of a mutations to a polynucleotide or polypeptide sequence refers to an *a priori* specified, selected, and/or desired change to the sequence

(*e.g.,* a nucleotide change from c to g in a selected position of a polynucleotide sequence that results in a different amino acid at a desired position of the encoded polypeptide).

"Deletion" with respect to a polypeptide or polynucleotide refers to the removal of one or more amino acids or nucleotides from the reference polypeptide or polynucleotide, respectively. Deletions can comprise removal of 1 or more amino acids or nucleotides, 2 or more amino acids or nucleotides, 3 or more amino acid or nucleotides, 5 or more amino acids, 6 or more amino acids or nucleotides, 10 or more amino acids or nucleotides, 15 or more amino acids or nucleotides, or 20 or more amino acids or nucleotides, up to 10% of the total number of amino acids or nucleotides, or up to 20% of the total number of amino acids or nucleotides making up the reference polypeptide or polynucleotide. Deletions can be directed to the internal portions and/or terminal portions of the polypeptide or polynucleotide. In various embodiments, the deletion can comprise a continuous segment or can be discontinuous.

"Heterologous" when used with reference to a nucleic acid or polypeptide, indicates that a sequence that comprises two or more subsequences which are not found in the same relationship to each other as normally found in nature, or is recombinantly engineered so that its level of expression, or physical relationship to other nucleic acids or other molecules in a cell, or structure, is not normally found in nature. For instance, a heterologous nucleic acid is typically recombinantly produced, having two or more sequences from unrelated genes arranged in a manner not found in nature; e.g., a nucleic acid open reading frame (ORF) of the invention operatively linked to a promoter sequence inserted into an expression cassette, e.g., a vector.

"Insertion" or "Addition" refers to a change in a nucleotide or amino acid sequence by the addition of one or more nucleotides or amino acid residues, respectively, as compared to a reference sequence, such as for example, a wild type sequence.

"Library" refers to a set (e.g., a plurality) of heterogeneous polypeptides or nucleic acids. A library is composed of members, which have a single polypeptide or nucleic acid sequence. To this extent, "library" is synonymous with "repertoire". Sequence differences between library members are responsible for the diversity present in the library. The library may take the form of a simple mixture of polypeptides or nucleic acids, or may be in the form organisms or cells, for example bacteria, viruses, animal or plant cells and the like, transformed with a library of nucleic acids.

"Non-conservative substitution" refers to substitution of an amino acid in the polypeptide with an amino acid with significantly differing side chain properties. Non-conservative substitutions may use amino acids between, rather than within, the defined groups and affects (a) the structure of the peptide backbone in the area of the substitution (*e.g.,* proline for glycine) (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

"Naturally-occurring" or "wild-type" refers to the form found in nature. For example, a naturally occurring or wild-type polypeptide or polynucleotide sequence is a sequence present in an organism that can be isolated from a source in nature and which has not been intentionally modified by human manipulation.

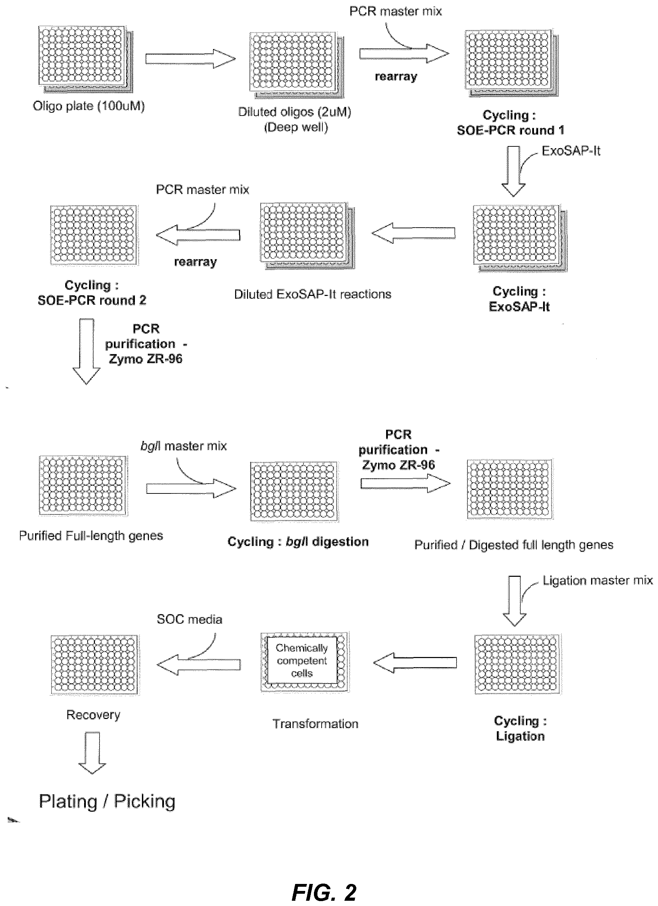

"Nucleobase" or "base" refers to those naturally occurring and synthetic heterocyclic moieties commonly known to those who utilize nucleic acid or polynucleotide technology or utilize polyamide or peptide nucleic acid technology to thereby generate polymers that can hybridize to polynucleotides in a sequence-specific manner. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine). Other non-limiting examples of suitable nucleobases include those nucleobases illustrated in Figures 2(A) and 2(B) of Buchardt et al. (WO 92/20702 or WO 92/20703).

"Nucleobase Polymer" or "Oligomer" refers to two or more nucleobases that are connected by linkages that permit the resultant nucleobase polymer or oligomer to hybridize to a polynucleotide having a complementary nucleobase sequence. Nucleobase polymers or oligomers include, but are not limited to, poly- and oligonucleotides (e.g., DNA and RNA polymers and oligomers), poly- and oligonucleotide analogs and poly- and oligonucleotide mimics, such as polyamide or peptide nucleic acids. Nucleobase polymers or oligomers can vary in size from a few nucleobases, from 2 to 40 nucleobases, to several hundred nucleobases, to several thousand nucleobases, or more.

"Operably linked" refers to a functional relationship between two or more nucleic acid (e.g., DNA) segments. In some embodiments, it refers to the functional relationship of a transcriptional regulatory sequence to a transcribed sequence. For example, a promoter (defined below) is operably linked to a coding sequence, such as a nucleic acid of the invention, if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance. In some embodiments, regulatory sequence is a translation regulatory sequence linked to a coding sequence.

"Overlapping region" refers to a region of a first polynucleotide that is complementary to a second polynucleotide, where the overlapping regions are capable of annealing to each other to form a hybridization complex. Generally, the first polynucleotide and the second polynucleotide will partially overlap such that the polynucleotides will have non-complementary regions that do not anneal between the two polynucleotides.

"Permutations" refers to the arrangement of elements (e.g., substitution mutations) from a given finite set. In the context of the descriptions herein for polypeptides and polynucleotides, differences in amino acid residues or nucleotide residues from a reference sequence, typically characterized as "mutations", can be arranged in various combinations in the sequence to form a permutation of the mutation set. Permutations include single mutations as well every combination of mutations possible from the defined set.

"Polynucleotides" or "Oligonucleotides" refer to nucleobase polymers or oligomers in which the nucleobases are connected by sugar phosphate linkages (sugar-phosphate backbone). Exemplary poly- and oligonucleotides include polymers of 2' deoxyribonucleotides (DNA) and polymers of ribonucleotides (RNA). A polynucleotide may be composed entirely of ribonucleotides, entirely of 2' deoxyribonucleotides or combinations thereof.

"Polynucleotide" or "Oligonucleotide Analog" refers to nucleobase polymers or oligomers in which the nucleobases are connected by a sugar phosphate backbone comprising one or more sugar phosphate analogs. Typical sugar phosphate analogs include, but are not limited to, sugar alkylphosphonates, sugar phosphoramidites, sugar alkyl- or substituted alkylphosphotriesters, sugar phosphorothioates, sugar phosphorodithioates, sugar phosphates and sugar phosphate analogs in which the sugar is other than 2'-deoxyribose or ribose, nucleobase polymers having positively charged sugar-guanidyl interlinkages such as those described in U.S. Patent No. 6,013,785 and U.S. Patent No. 5,696,253 (see also, Dagani 1995, Chem. Eng. News 4-5:1153; Dempey et al., 1995, J Am Chem Soc 117:6140-6141). Such positively charged analogues in which the sugar is 2'-deoxyribose are referred to as "DNGs," whereas those in which the sugar is ribose are referred to as "RNGs." Specifically included within the definition of poly- and oligonucleotide analogs are locked nucleic acids (LNAs; see, e.g. Elayadi et al., 2002, Biochemistry 41:9973-9981; Koshkin et al., 1998, J Am Chem Soc 120:13252-3; Koshkin et al., 1998, Tetrahedron Letters 39:4381-4384; Jumar et al., 1998, Bioorganic & Medicinal Chemistry Letters 8:2219-2222; Singh and Wengel, 1998, Chem. Commun., 12:1247-1248; WO 00/56746; WO 02/28875; and, WO 01/48190).

"Primers" refer to oligonucleotides having sequence complementary to a target sequence, generally referred to as a primer binding sequence. The complementary portion of a primer can be any length that supports specific and stable hybridization between the primer and the target sequence under the reaction conditions. The primers can be about 5 to 60 nucleotides long, about 10 to 35 nucleotides long, or can be from, and in particular, 10, 11, 12, 13, 14, 15, 16, 17, 18, 19, and/or 20 nucleotides long. Generally, primers for replication by polymerase are capable of supporting extension by the polymerase when the primer is annealed to the target sequence. "Amplification primer" refers to an oligonucleotide primer used for amplification of a target nucleic acid sequence.

"Forward primer" and "reverse primer" refer to a set of amplification primers, where one primer anneals to the 3' end of the target (template strand) while the other primer anneals to the 3' end of the complementary target strand to amplify an amplicon.

"Proximate" refers to the nucleotide distance from a defined base (e.g., a first nucleotide mutation) to a second defined base (e.g., a second nucleotide mutation), where the first and second mutations can be accommodated in a single oligonucleotide primer used for amplification purposes (e.g., forward or reverse primer). Thus, in some embodiments, the term "proximate" is determined with respect to the length of the primer. In some embodiments, two mutations can be proximate if they are separated by 1, 2, 3, 4, 5, 6, 8, 10, 12, 14, 16, 18, 20, or 25 nucleotide bases and are within the primer. In some embodiments, the location of the mutations with respect to the 3' end of the primer is such that an oligonucleotide annealed to a template strand can undergo extension by polymerase, as described in detail below.

"Protein," "polypeptide," "oligopeptide," and "peptide" are used interchangeably to denote a polymer of at least two amino acids covalently linked by an amide bond, regardless of length or post-translational modification (e.g., glycosylation, phosphorylation, lipidation, myristilation, ubiquitination, etc.). Included within this definition are D- and L-amino acids, and mixtures of D- and L-amino acids.

"Recombinant" when used with reference to, e.g., a cell, nucleic acid, polypeptide, expression cassette or vector, refers to a material, or a material corresponding to the natural or native form of the material, that has been modified by the introduction of a new moiety or alteration of an existing moiety by recombinant techniques, or is identical thereto but produced or derived from synthetic materials using recombinant techniques. For example, recombinant cells express genes that are not found within the native (non-recombinant) form of the cell (i.e., "exogenous nucleic acids") or express native genes that are otherwise expressed at a different level, typically, under-expressed or not expressed at all.

"Reference sequence" refers to a defined sequence used as a basis for a sequence comparison. A reference sequence may be a subset of a larger sequence, for example, a segment of a full-length gene or polypeptide sequence. Generally, a reference sequence is at least 20 nucleotide or amino acid residues in length, at least 25 residues in length, at least 50 residues in length, or the full length of the nucleic acid or polypeptide. Since two polynucleotides or polypeptides may each (1) comprise a sequence (i.e., a portion of the complete sequence) that is similar between the two sequences, and (2) may further comprise a sequence that is divergent between the two sequences, sequence comparisons between two (or more) polynucleotides or polypeptides are typically performed by comparing sequences of the two polynucleotides or polypeptides over a "comparison window" to identify and compare local regions of sequence similarity.

"Replicating" refers to copying of a target polynucleotide sequence to synthesize a reverse complementary copy of the polynucleotide. Generally, replicating is done by polymerases that copy a template polynucleotide to synthesize a polynucleotide that is a reverse complement of the target polynucleotide sequence.

"Segment" refers to a sequence that is a portion of a larger polynucleotide sequence. The larger polynucleotide sequence can be divided into a plurality of segments, wherein the combination of the segments comprises the full length of the larger polynucleotide sequence.

"Polypeptide variant" or " polypeptide analog" as used herein refers to polypeptides which are comprised of a segment having functional activity, with or without retention of any improved property, and has substantial identity to a portion of a reference polypeptide. In some embodiments, analog polypeptides comprise a conservative or non-conservative amino acid substitution, or addition or deletion of one or more amino acid residues with respect to the reference sequence.

"Watson/Crick Base-Pairing" refers to a pattern of specific pairs of nucleobases and analogs that bind together through sequence-specific hydrogen-bonds, e.g., A pairs with T and U, and G pairs with C.

"Substitution" refers to the replacement of one or more nucleotides or amino acids by different nucleotides or amino acids, respectively, with respect to a reference sequence, such as, for example, a wild-type sequence.

"Substrate," "Support," "Solid Support," "Solid Carrier," or "Resin" are interchangeable terms and refer to any solid phase material. Substrate also encompasses terms such as "solid phase," "surface," and/or "membrane." A solid support can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. The configuration of a substrate can be in the form of beads, spheres, particles, granules, a gel, a membrane or a surface. Surfaces can be planar, substantially planar, or non-planar. Solid supports can be porous or non-porous, and can have swelling or non-swelling characteristics. A solid support can be configured in the form of a well, depression, or other container, vessel, feature, or location. A plurality of supports can be configured on an array at various locations, addressable for robotic delivery of reagents, or by detection methods and/or instruments.

"Array" refers to an arrangement of agents (e.g., proteins, antibodies, replicable genetic packages) on a substrate in positionally distinct locations. In some embodiments, the agents on the array are spatially encoded such that the identity of an agent can be determined from its location on the array. A "microarray" generally refers to an array in which detection requires the use of microscopic detection to detect complexes formed with agents on the substrate. A "location" on an array refers to a localized area on the array surface that includes agents, each defined so that it can be distinguished from adjacent locations (e.g., being positioned on the overall array, or having some detectable characteristic, that allows the location to be distinguished from other locations). The location can have any convenient shape (e.g., circular, rectangular, elliptical or wedge-shaped). The size or area of a location can vary significantly. Arrays may be constructed on substrates, such as glass or plastic microscope slides, and be configured in the form of wells, depressions, droplets, or other containers, or reaction vessels, such as a microplate well. In general, there is no restriction on the format of the array provided the individual sites to which the agents are disposed can be localized and identified.

"Reaction chamber" is meant the environment in which agents and/or reaction components takes place. Commercially available reaction vessels contain at least one reaction chamber, but can contain 8, 24, 96 or 384 reaction chambers. For the purposes of the present disclosure, "reaction chamber(s)," "well(s)," "reaction site(s)," are used interchangeably. An example of a reaction chamber is one of the 96 microtiter wells in a 96 well microtiter plate.

"Primer array" refers to the array of primers or primer sets used in an amplification reaction in positionally distinct locations on a substrate (e.g., array substrate). Generally, the primer set comprises a pair of forward and reverse primers used to amplify an amplicon.

"Amplicon array" refers to an arrangement of amplified polynucleotides in positionally distinct locations on a substrate (e.g., array substrate). In some embodiments, the amplicon array can have the same positional arrangement as the primer array, such as when the amplification reaction is carried out in the primer array to generate amplified polynucleotides.

"Plasmid", "vector", and "cassette" refer to an extra chromosomal element often carrying genes, and usually in the form of circular double-stranded DNA molecules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. "Expression cassette" refers to a specific vector containing a foreign gene and having elements in addition to the foreign gene that allow for expression of that gene in a host.

### 4.2 Methods of Synthesizing Polynucleotide Variants

The present disclosure provides methods of generating polynucleotide variants having a defined set of sequence differences from a reference polynucleotide sequence. In some embodiments, the methods are applicable for generating polynucleotides encoding polypeptide variants having defined differences in amino acid sequence as compared to a reference polypeptide. In some embodiments, the polynucleotide variants have a defined set of nucleotide differences in non-coding regions, e.g., silent mutations. The polynucleotides are efficiently generated by using libraries of polynucleotide fragments, where the members of the library encode one or more of the amino acid differences as compared to a reference polypeptide sequence, and the polynucleotide fragments are designed to have overlapping adjacent regions such that selection of an appropriate set of fragments, with and without mutations, allows their assembly into a polynucleotide variant, such as a polynucleotide encoding a desired polypeptide variant.

In some embodiments, the method for generating a polynucleotide encoding a polypeptide having an amino acid sequence with one or more defined differences in amino acid residues, comprises (a) selecting a plurality of defined amino acid residue differences relative to a reference amino acid sequence; (b) defining overlapping segments of a polynucleotide sequence encoding the polypeptide with the different amino acid sequence, or optionally the reference polypeptide, with each segment being bounded by a set of forward and reverse primer binding sequences, wherein a polynucleotide sequence difference encoding each of the plurality of amino acid residue differences is encompassed in the sequences of the forward and/or reverse primers that bind to the primer binding sequences; (c) amplifying each segment with the set of forward and reverse primers, wherein selected forward and/or reverse primer contain the polynucleotide sequence differences, to generate a library of amplicons comprising members encoding the defined amino acid differences and wherein the library comprises members sufficient for assembling two or more different amino acid sequence permutations of the defined amino acid residue differences; (d) assembling from the library a set of amplicons having complementary adjacent regions that together encode the polypeptide with a defined amino acid sequence permutation having one or more defined amino acid residue differences; and (e) replicating the set of assembled amplicons to synthesize the polynucleotide encoding the polypeptide. A library of amplicons containing all of the defined amino acid differences should allow the synthesis of a plurality of polynucleotides that encode for all possible permutations of amino acid sequences.

As will be apparent to the skilled artisan, selecting the plurality of defined amino acid residue differences can be obtained from various sources. In some embodiments, the amino acid residue positions and corresponding mutations for a defined polypeptide can be obtained from studies of random mutagenesis, such as that described in Crameri et al., 1998, "DNA shuffling of a family of genes from diverse species accelerates directed evolution," Nature 391:288-291; Crameri et al., 1997, "Molecular evolution of an arsenate detoxification pathway by DNA shuffling," Nature Biotech 15:436-438; Zhang et al., 1997, "Directed evolution of an effective fructosidase from a galactosidase by DNA shuffling and screening," Proc Natl Acad Sci USA 94:45-4-4509; Crameri et al., 1996, "Improved green fluorescent protein by molecular evolution using DNA shuffling, Nature Biotech 14:315-319; Stemmer, 1994, "Rapid evolution of a protein in vitro by DNA shuffling," Nature 370:389-391; Stemmer, 1994, "DNA shuffling by random fragmentation and reassembly: In vitro recombination for molecular evolution," Proc Natl Acad Sci USA 91:10747-10751; WO 95/22625; WO 97/0078; WO 97/35966; WO 98/27230; WO 00/42651; WO 01/75767 and U.S. Pat. 6,537,746.

Typically, a mutagenized library of polynucleotides is expressed and the expressed polypeptides screened for a desired property trait, and the mutations associated with the changes in the desired property identified. Large number of mutations affecting a polypeptide function can be readily obtained using these techniques.

In some embodiments, the choices of amino acid residue differences can be obtained from comparison of amino acid sequences of related proteins, such as those found in a sequence database. Sequence comparison can identify positions, for example conserved residues that may be important to protein function, which can then be targeted for defined amino acid changes. See, e.g., Wankhade et al., 2000, J. Biol. Chem. 275(38):29701-29708 and Reddy et al., 2001, Proteins: Structure, Function, and Genetics 42:148-163.

In some embodiments, the plurality of defined amino acid residue differences can be based on sequence differences found in nature, such as polymorphisms found for a particular gene. In some instances, the polymorphisms are associated with particular biological effects and associated phenotypes. See, e.g., Bidwell et al., 1999, Genes and Immunity 1:3-19; Chen et al., 2003, Mol. Biol. Evo. 18:1771-1788. Collections of polymorphisms can form the basis for defining a plurality of the amino acid residue differences for forming variant polypeptides of a reference amino acid sequence. Combinations of different amino acid polymorphisms can be used to examine the function of a particular protein.

When the plurality of amino acid residue differences is defined with respect to a reference sequence, the polynucleotide encoding the reference polypeptide or the polypeptide variants can be used as a basis for identifying segments for defining the amplicons to be generated to create the polynucleotide fragment (i.e., amplicon) library. In some embodiments, the polynucleotide sequence need not be restricted to any particular sequence as long as it encodes, or can be used as a basis to generate polynucleotides that encode, the amino acid sequence of interest. The polynucleotide can be based on the naturally occurring (e.g., wild-type) sequence or a sequence optimized for expression in a particular organism of interest (e.g., codon optimized). For example, if the polypeptide of interest is to be expressed in *E. coli.,* a polynucleotide sequence in which the codons are optimized for expression in *E. coli.* can be used. Codon optimization techniques are well within the skill of those in the art.

As will be apparent to the skilled artisan, dividing the polynucleotide into defined segments for amplification can be accomplished using techniques well known in the art. In some embodiments, since the segments are defined by primer binding sequences, which are themselves used to introduce mutations into the amplicon, division of the polynucleotide into segments can initially take into account the location of the mutations on the polynucleotide. The divisions of the polynucleotide into segments can also take into account the total length of the polynucleotide, the efficiency of replication (e.g., amplification of segments), and the desired number of amplicons for assembly. Other considerations will be apparent to the skilled artisan.

Amplification reactions can be affected by sequence, type of polymerase used, efficiency of primers, and unwanted side reactions (e.g., primer dimers). Thus, in some embodiments, depending on the total length of the polynucleotide to be assembled, the segment lengths can be 2000 bases or less, 1500 bases or less, 1200 bases or less, 1000 bases or less, 900 bases or less, 800 bases or less, 700 bases or less, 600 bases or less, 500 bases or less, 400 bases or less, 300 bases or less, 250 bases or less, or 200 bases or less to about 100 or as few as about 50 bases in length. Generally, length of the segments is from about 50 to about 1000 bases, about 200 to 1000 bases, about 300 to 700 bases, or about 400 to 600 bases, with about 500 bases being useful average length given the efficiency of polymerases used in amplification reactions. In various embodiments, the segments are overlapping such that the amplicons produced therefrom will also have overlapping adjacent regions (i.e., overlapping complementary regions) for assembling the polynucleotide.

In some embodiments, the adjacent overlapping regions should be of sufficient length and complementarity to permit the formation of stable annealed (i.e., hybridized) amplicons during assembly of the polynucleotide. Thus, in some embodiments, the length of overlap can be 4 or more nucleotides, 5 or more nucleotides, 6 or more nucleotides, 8 or more nucleotides, 10 or more nucleotides, 15 or more nucleotides, 20 or more nucleotides, 25 or more nucleotides, 30 or more nucleotides, 40 or more nucleotides, 50 or more nucleotides, and 100 or less, 90 or less, 80 or less, 70 or less, 60 or less nucleotides in length as permitted by the ability to form stable annealed amplicons. Since the overlap regions generally include the primer binding sequences use to generate the amplicons, the length of overlap can account for any differences in the sequence of the primer (e.g., forward and/or reverse) used to generate the polynucleotide differences encoding the mutation to be introduced.

In some embodiments, the segments are bounded by primer binding sequences to which the forward/reverse primers anneal. Where appropriate, the primer binding sequences that define the segments can also encompass the position of the polynucleotide that encodes an amino acid sequence difference. The primer binding sequence can be of any sufficient length to anneal to the primer (forward or reverse) during the amplification reaction. Accordingly the primer binding sequence can be 100 bases or less, 90 bases or less, 80 bases or less, 70 bases or less, 60 bases or less, 50 bases or less, 40 bases or less, 30 bases or less, 20 bases or less 15 bases or less, to about 8 bases or 10 bases. In some embodiments, the length of the primer binding sequences can comprise from about 8 to 50 bases, about 8 to 40 bases, about 10 to 30 bases, or about 15 to 25 bases. The primers typically can comprise lengths complementary to the primer binding sequences described above. Accordingly, in some embodiments, the length of the forward/reverse primers can be about 60 nucleotides or less, 50 nucleotides or less, 40 nucleotides or less, 30 nucleotides or less, 20 nucleotides or less 15 nucleotides or less, to about 10 nucleotides or even 8 nucleotides. In some embodiments, the length of the forward/reverse primers can be from about 8 to 50 nucleotides, about 8 to 40 nucleotides, about 10 to 30 nucleotides, or about 15 to 25 nucleotides.

Where the primer contains a sequence encoding a defined amino acid difference, the mutation can be located at a region of the primer that does not interfere with primer extension. In some embodiments, the mutation is located at about the middle of the mutagenic primer, where the primer has a Tm that is sufficient to anneal to the template nucleic acid and serve as a primer for the polymerase mediated extension reaction. In some embodiments, the polynucleotide sequence differences can be located, depending on the length of the primer, about 5 bases, 6 bases, 8 bases, 10 bases, 12 bases, 15 bases, 20 bases, 25 bases from the 3' end of the primer. Accordingly, in some embodiments the length of the forward/reverse primers can be from about 8 to 50 nucleotides, about 8 to 40 nucleotides, about 10 to 30 nucleotides, or about 15 to 25 nucleotides, and further comprise nucleotide sequence difference at about the middle of the primer. Thus, in some embodiments the forward/reverse primers are about 50 nucleotides in length with a nucleotide difference about 25 nucleotides from the 3' end, about 40 nucleotides in length with a nucleotide difference about 20 nucleotides from the 3' end, about 30 nucleotides in length with a nucleotide difference about 15 nucleotides from the 3' end, about 25 nucleotides in length with a nucleotide difference about 12 nucleotides from the 3' end, or about 20 nucleotides in length with a nucleotide difference about 10 nucleotides from the 3' end.

The stability of the oligonucleotide primers, e.g., the thermal melting temperature, is a function of ion strength, temperature, G/C content, and the presence of chaotropic agents and can be calculated using known methods for predicting melting temperatures (see, e.g., Baldino et al., Methods Enzymology 168:761-777; Bolton et al., 1962, Proc. Natl. Acad. Sci. USA 48:1390; Bresslauer et al., 1986, Proc. Natl. Acad. Sci USA 83:8893-8897; Freier et al., 1986, Proc. Natl. Acad. Sci USA 83:9373-9377; Kierzek et al., Biochemistry 25:7840-7846; Rychlik et al., 1990, Nucleic Acids Res 18:6409-6412 (erratum, 1991, Nucleic Acids Res 19:698); Sambrook et al., 2001, Molecular Cloning: A Laboratory Manual, 3rd Ed., Cold Spring Harbor Laboratory Press, NY; Suggs et al., 1981, In Developmental Biology Using Purified Genes (Brown et al., eds.), pp. 683-693, Academic Press; and Wetmur, 1991, Crit Rev Biochem Mol Biol 26:227-259).

To generate the library of amplicons, forward and reverse primers that anneal to the primer binding sequences of each segment of the polynucleotide are used in an amplification reaction to generate amplicons. Where the amplicon has a polynucleotide difference encoding a defined amino acid change relative to the reference sequence, the sequence of the forward and/or reverse primers are designed to introduce the different sequence (*i.e.,* mutation) in the amplification reaction. Suitable combinations of forward and reverse primers are used to generate a library of amplicons comprising members that can encode for each of the plurality of amino acid residue differences.

In some embodiments, the sets of forward and reverse primers can be stored in an array, for example a primer array, such that they can be easily accessed when amplicons are needed for synthesis of a polynucleotide encoding a defined amino acid sequence permutation. As will be appreciated in the art, the oligonucleotide primers can be used to introduce any type of mutation selected in the defined plurality of amino acid residue differences, including, among others, amino acid insertions, deletions, and substitutions. The substitutions can be conservative or non-conservative mutations, as dictated by the chosen plurality of amino acid residue differences.

In some embodiments, more than one amino acid sequence difference can be present on the same amino acid residue position in a polypeptide sequence. In these embodiments, different amplicons from the same overlapping segment can be generated, where each amplicon is prepared with forward and reverse primer pairs for each defined mutation at the identical residue position. To prepare a polynucleotide encoding a particular sequence permutation at that specific amino acid residue position, one of the amplicons containing the desired mutation (*i.e.,* defined nucleotide difference) is chosen and assembled as a member of the set of amplicons to generate the polynucleotide encoding a polypeptide containing the desired mutation(s) at the specified amino acid residue position.

In some embodiments, more than one pair of primers (*e.g.,* a set of degenerate primers) can be used to generate a set of amplicons (*i.e.,* polynucleotide fragments) that can be used to assemble a set of polynucleotide variants encoding polypeptides having more amino acid residue changes (*e.g.,* substitutions) at a specific defined position. The polynucleotide variants assembled from the amplicons made using degenerate primers can be sequenced before or after their encoded polypeptide is assayed in order to determine the specific sequence at the position of interest.

As will be apparent to the skilled artisan, in some embodiments, an overlapping segment defined for a polynucleotide sequence may not have any associated mutations. Additionally, the same segment may in one amino acid sequence permutation encompass a specified mutation, but in some sequence permutations may not have any mutation associated with the segment. Thus in some embodiments, the library of amplicons can contain members that do not have any polynucleotide sequence differences as compared to the reference sequence for a particular segment. These bridging polynucleotides, which have no associated changes in sequence as compared to the reference sequence, can be used as a connector to assemble the complete polynucleotide.

With the appropriate choice of segments, the amplicon library comprises members that can be used to assemble at least two or more different amino acid sequence permutations of the defined amino acid differences relative to the reference sequence. For example, a plurality of mutations defined by amino acid residue differences A and B can have the following permutations: A alone, B alone, or A and B. Thus the amplicon library has sufficient members to generate an amino acid sequence permutation having independently an A mutation or B mutation. In some embodiments, the amplicon library has members sufficient to generate every amino acid sequence permutation of the defined amino acid residue differences relative to the reference sequence. Thus, for the given example, the amplicon library has sufficient members to generate amino acid sequence permutations having independently an A mutation or B mutation, or an A + B mutation. Since the size of the amplicons will correspond to the size of the segments, the amplicons can be 2000 bases or less, 1500 bases or less, 1200 bases or less, 1000 bases or less, 900 bases or less, 800 bases or less, 700 bases or less, 600 bases or less, 500 bases or less, 400 bases or less, 300 bases or less, 250 bases or less, or 200 bases or less to about 100 or as few as about 50 bases in length. Generally, length of the amplicons is from about 50 to about 1000 bases, about 200 to 1000 bases, about 300 to 700 bases, or about 400 to 600 bases, with about 500 bases or less a useful length given the efficiency of polymerases used in amplification reactions. In some embodiments, the amplicons are about 400 bases or less in length.

Generally, the amplification reaction can use any enzyme used for polymerase mediated extension reactions, such as Taq polymerase, Pfu polymerase, Pwo polymerase, Tfl polymerase, rTth polymerase, Tli polymerase, Tma polymerases, and Klenow fragment. Conditions for amplifying a polynucleotide segment using polymerase chain reaction can follow standard conditions known in the art. See, e.g., Sambrook et al., 2001, Molecular Cloning: A Laboratory Manual, 3rd Ed., Cold Spring Harbor Laboratory Press, NY and Ausubel et al., 1989, Current Protocols in Molecular Biology, Greene Publishing Associates and Wiley Interscience, N.Y (updates up to 2008).

In some embodiments, amplification of each amplicon can be carried out in separate reactions, thereby minimizing the need to isolate one amplicon product from another amplicon. However, the amplification reactions for two or more amplicons can be carried out in a single reaction and the products isolated, such as by electrophoresis or chromatography. In some embodiments, the products of the amplification reaction can be treated with various combinations of exonucleases and phosphatases to remove remaining primers and free nucleotides (e.g., combination of exonuclease I and alkaline phosphatase).

To generate the polynucleotide encoding the polypeptide with the defined amino acid sequence permutation, a set of amplicons having complementary overlapping regions is selected and assembled under conditions that permit the annealing of the complementary overlapping regions to each other. For example, the amplicons can be denatured and then allowed to anneal to form a complex of amplicons that together encode the polypeptide with a defined amino acid sequence permutation having one or more of the amino acid residue differences relative to a reference sequence. Generally, assembly of each set of amplicons can be carried out separately such that the polynucleotide encoding one amino acid sequence permutation is readily distinguished from another polynucleotide encoding a different amino acid sequence permutation. In some embodiments the assembly can be carried out in addressable locations on a substrate (e.g., an array) such that a plurality of polynucleotides encoding a plurality of defined amino acid sequence permutations can be generated simultaneously.

In some embodiments, assemblies can be prepared such that multiple (*i.e.,* 2 or more) amplicons are represented for the same fragment. The resulting product from this assembly reaction will contain a mixture of polynucleotides containing different permutations of the defined amino acid sequence differences. This mixture can be cloned directly and variants can be sequenced before or after encoded polypeptides are assayed.

The assembled amplicons is replicated using a polymerase to synthesize the polynucleotide encoding the polypeptide of interest. In some embodiments, the reaction conditions can use the same conditions and polymerases used for the amplification reaction. The assembled amplicons act as primers such that a single round of replication creates a duplicate of the assembled amplicons. Generally, in the replicating step, primers that anneal to primer binding sequences that flank the polynucleotide (i.e., terminal 5' region and terminal 3' region) can be added to amplify the polynucleotide product by carrying out additional amplification reactions. In some embodiments, these flanking primers can incorporate recognition sequences for restriction enzymes to ease cloning of the synthesized polynucleotide product into plasmids or vectors, such as expression vectors.

In some embodiments, the flanking primers can have sequences that allow for direct *in vitro* expression using a coupled transcription-translation systems for synthesis of the protein product without the need for transformation into a host organism. Hence, some flanking primers can incorporate control sequences to control the expression of the polypeptide coding region. Amplification reactions using such flanking primers can operably link the control sequences to the polypeptide coding region of interest.

In some embodiments, the plurality of amino acid differences is at least 2. In some embodiments, the plurality of amino acid differences is at least 2, 3, 4, 5, 6, 7, 8, 9, 10, 11, 12, 13, 14, 15, or more. Accordingly, the number of defined nucleotide differences can range from 2 to 45, or more. The number of permutations for "n" plurality of defined amino acid residue difference is given by the formula n!/(k!(n-k)!, where n is the number of non-mutually exclusive mutations and k is the number of amino acid differences and n! denotes the factorial operator. In some embodiments, the size of the amplicon library, for example for a minimum of 2 amino acid residue differences is a library size containing at least 3 different amplicons. In some embodiments, the size of the library is at least 5, 6, 7, 8, 9, 10, 11, or even more different amplicons. For example, for a plurality of variants comprising at least 10 defined differences, assuming none of the differences are located proximately such that more than 1 can be included per primer, assembling variants with 10 defined differences uses up to 11 amplicons per assembly reaction. Assuming a plurality of different mutations are desired at any of the plurality of positions having defined differences, much larger libraries of amplicons can be used. Accordingly, in some embodiments, the library of amplicons can comprise is at least 5, 10, 20, 30, 40, 50, 75, 100, or more different amplicons.

Once the library of amplicons have been synthesized, any polynucleotide encoding a specified amino acid sequence permutation based on a plurality of amino acid residue difference can be made using the library of amplicons. In some embodiments, the method of generating a polynucleotide encoding a polypeptide having an amino acid sequence with one or more defined differences in amino acid residues as compared to a reference polypeptide sequence can comprise the steps of: (a) assembling a set of amplicons having complementary overlapping adjacent regions, where the assembled set of amplicons comprise a polynucleotide sequence encoding an amino acid sequence with one or more defined amino acid residue difference as compared to a reference sequence, where the amplicons are selected from a library of amplicons having members encoding a plurality of amino acid differences, and (b) replicating the set of assembled overlapping polynucleotide fragments to synthesize the polynucleotide of interest.

In some embodiments, the amplicon library can be used to generate polynucleotides encoding any permutation of a defined plurality of defined amino acid differences, the method comprising: (a) generating permutations of amino acid sequences differing from a reference amino acid sequence based on a plurality of defined amino acid residue differences as compared to a reference amino acid sequence, (b) selecting a defined amino acid sequence permutation and determining a corresponding polynucleotide sequence based on a reference sequence, (c) selecting a set of overlapping polynucleotide fragments encoding the defined amino acid sequence permutations, where at least each overlapping polynucleotide fragment encoding an amino acid difference is from a plurality of polynucleotide fragments encoding different known amino acid residue differences, wherein the plurality of fragments has members sufficient to assemble polynucleotides encoding at least two different amino acid sequence permutations, (d) assembling the set of polynucleotide fragments having complementary overlapping adjacent regions, and (e) replicating the set of assembled overlapping fragments to synthesize the polynucleotide encoding the polypeptide. For each desired amino acid sequence permutation, the steps of (b) to (e) can be repeated.

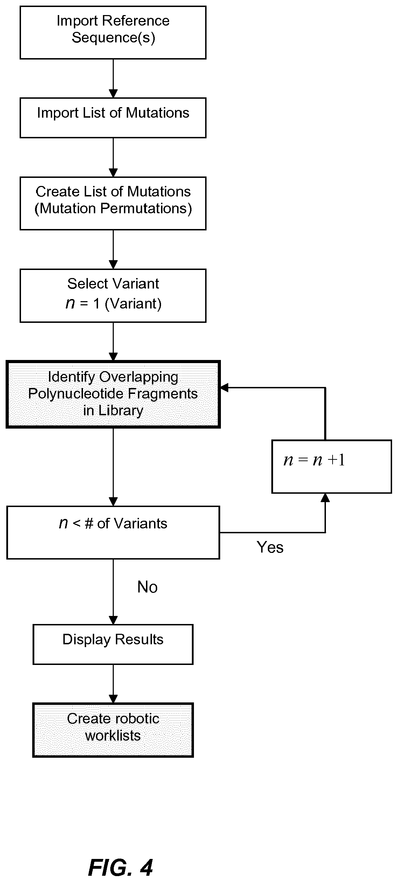

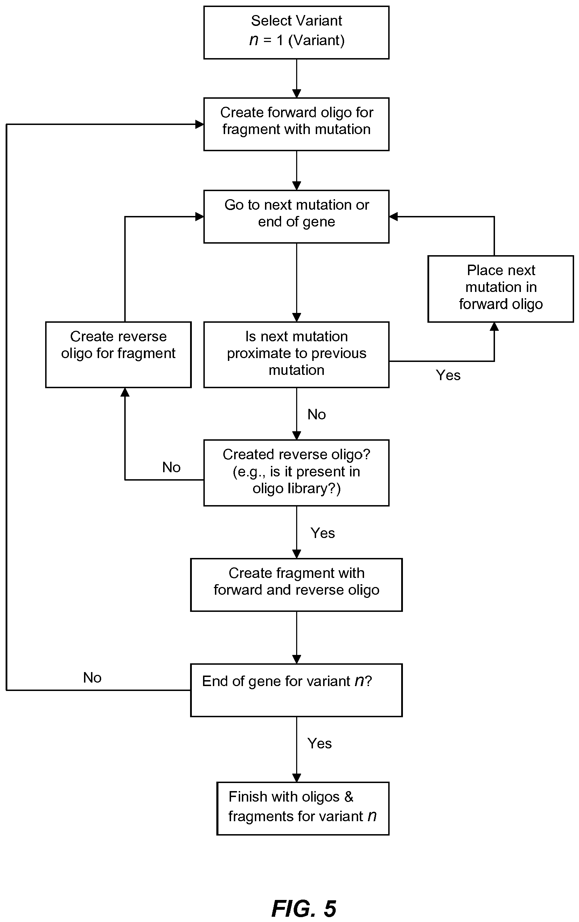

An exemplary process for generating the amplicons for "n" number of variants is shown in **FIG. 4****.** In the illustrated embodiment, the process comprises: (a) importing a reference sequence and a list of mutations associated with the sequence, (b) creating a list of permutations based on the list of mutations, (c) selecting a defined permutation of the amino acid sequence (i.e., variant 1), (d) identifying overlapping polynucleotide fragments from a library of amplicons (e.g., as prepared in **FIG. 5**), (e) determining the number of variants and if the number of variants is less than the total number of desired variants, reiterating steps (a) to (d).

For efficient synthesis of the amplicon libraries, appropriately designed oligonucleotide primers are used in an amplification reaction. In some embodiments, the method of generating a library of overlapping polynucleotide fragments can comprise: (a) generating a plurality of permutations of amino acid sequences differing from a reference amino acid sequence based on a plurality of defined amino acid residue differences from a reference amino acid sequence, and for each permutation (i) determining a polynucleotide sequence encoding the amino acid sequence based on a reference polynucleotide sequence; (ii) scanning a polynucleotide sequence and identifying a change in polynucleotide sequence encoding an amino acid residue difference, and optionally determining the proximity of a next change in polynucleotide sequence encoding a next amino acid residue difference in the amino acid sequence permutation; (iii) selecting a forward oligonucleotide primer having a sequence encoding the amino acid difference, and optionally including the next change in polynucleotide sequence in the same forward primer if proximate to the change in polynucleotide sequence; (iv) scanning a polynucleotide sequence from the location of the forward primer until the next change in polynucleotide sequence is identified or until the end of the polynucleotide, and selecting a reverse oligonucleotide primer for amplifying a polynucleotide fragment with the forward oligonucleotide primer, wherein the reverse primer has a sequence that optionally encodes the next change in amino acid residue difference; (v) reiterating steps (ii) to (iv) for each change in polynucleotide sequence encoding an amino acid residue difference until all changes in polynucleotide sequence are present on oligonucleotide primers and ends of the polynucleotide sequence is reached; and (g) amplifying with each set of forward and reverse oligonucleotide primers to generate the library of overlapping amplicons having members encoding the amino acid differences. In these embodiments, when scanning of the polynucleotide sequence encounters the end of the polynucleotide, flanking primers can be used in combination with the internal primers to complete the generation of the amplicons.

An exemplary process for selecting the appropriate forward and reverse primers is illustrated in **FIG. 5**. In **FIG. 5****,** the process for selecting the oligonucleotide primers comprises: (a) selecting a variant (an amino acid sequence permutation) and generating its corresponding polynucleotide sequence based on a reference sequence, (b) creating a forward oligonucleotide primer for a fragment with a first mutation, (c) scanning the sequence from the first mutation to the next mutation or to the end of the gene and creating a reverse oligonucleotide primer for the next mutation, (d) and if the next mutation is proximate to the first mutation, placing the next mutation in the same forward oligonucleotide primer, (e) reiterating steps (b) to (d) until ends of polynucleotide variant n is reached.

As noted above, in some embodiments where the polynucleotide has been separated out into overlapping segments defined by a set of forward and reverse primers, the forward and reverse primers may have no associated mutations. One context in which this may occur is if the polynucleotide segments are to be restricted in size, for example about less than 1000 bases, because of a need for efficient synthesis of an amplicon, such that not all the segments have defined changes in polynucleotide sequence. In some embodiments, in preparing the oligonucleotides based on the method above, the search of the sequence can be limited to a particular size "1", for example by about 1200 bases in step (iv) for selecting a reverse primer. In other words, following the identification of a forward primer based on a sequence difference, a scan is made in one or the other direction of the polynucleotide sequence to determine the nucleotide distance to the next mutation. If the distance exceeds the set limit, a segment that does not encompass any mutations can be created to bridge two segments that contain the two distant mutations. The scanning process can be reiterated at the point of the next mutation.

As noted above, the oligonucleotide primers, either alone or in sets (e.g., forward and reverse oligonucleotides) as well as the corresponding amplicons can be placed on addressable substrates for automation and/or storage. Oligonucleotide primers in the addressable substrates, also described herein as primer array, can be robotically accessed to synthesize any libraries of amplicons for a defined plurality of amino acid differences. Likewise, the amplicons in the addressable substrates, also described herein as amplicon arrays, can be accessed to generate a polynucleotide sequence encoding a desired amino acid sequence permutation based on the defined plurality of amino acid residue differences. A substrate or solid support for the array can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. The configuration of a substrate can be in the form of beads, spheres, particles, granules, a gel, a membrane or a surface. Surfaces can be planar, substantially planar, or non-planar. Solid supports can be porous or non-porous, and can have swelling or non-swelling characteristics. A solid support can be configured in the form of a well, depression, or other container, vessel, feature, or location. A plurality of supports can be configured on an array at various locations, addressable for robotic delivery of reagents, or by detection methods and/or instruments. In some embodiments, the substrate is a reaction chamber. Commercially available reaction vessels contain at least one reaction chamber, but can contain 8, 24, 96 or 384 reaction chambers. An example of a reaction chamber is one of the 96 microtiter wells in a 96 well microtiter plate.

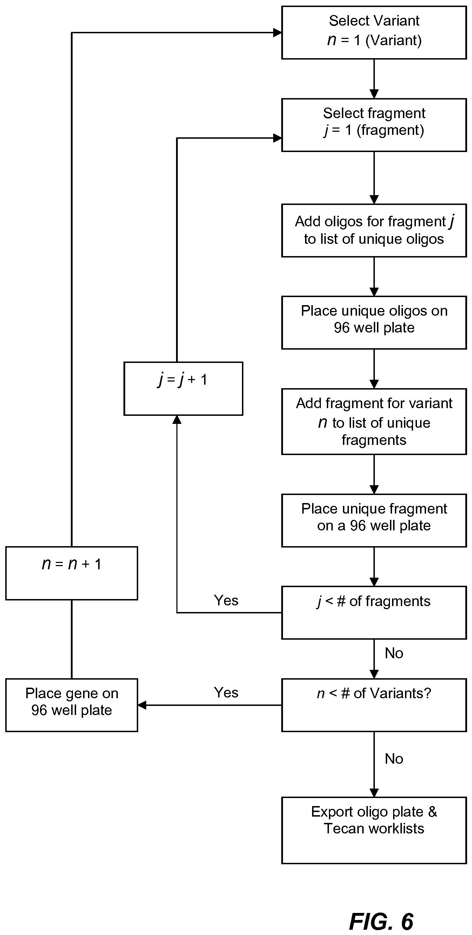

In some embodiments, a robotic system and an associated computer system capable of sampling primers or primer pairs from the arrays can be used to deliver them to a reaction chamber. Reagents for polymerase mediated amplification can also be delivered to each set of primers in the reaction chamber followed by implementation of an amplification routine (such as in a automated thermocycler). This allows formation of an addressable substrate containing defined amplicons based on overlapping segments of a polynucleotide sequence. The robotic system can choose the appropriate set of amplicons based on the desired permutation of the amino acid sequence, the flanking primers for amplification of the final polynucleotide product, and deliver the reagents for the assembly and amplification reaction. An exemplary robotic system is provided in **FIG. 6****.** The robotic system in **FIG. 6** comprises instructions for (a) selecting a segment and associated amplicon for amplification, (b) identifying forward and reverse oligonucleotides for the selected fragment (i.e., amplicon), storing data information on the oligonucleotides on list of unique oligonucleotides (e.g., 96 well microtiter plate), and placing the oligonucleotides on a first addressable substrate (c) storing data information on synthesized fragment (e.g., position on array, sequence, oligonucleotides used, etc) to list of unique fragments, and placing the oligonucleotide on a second addressable substrate, (d) determining the number of fragments selected against the total number of fragments required for assembly, and reiterating steps (a) to (d) until all fragments have been selected, (e) placing the assembled gene into a third addressable substrate, and reiterating steps (a) to (d) until all desired variants have been generated.

In some embodiments, the present disclosure also provides libraries of polynucleotide fragments (i.e., amplicons) for assembling a plurality of polynucleotides encoding different amino acid sequence permutations. In some embodiments, the plurality of polynucleotides comprises: polynucleotide fragments with overlapping adjacent regions, each polynucleotide fragment being bounded by primer binding sequences for forward and reverse primers, wherein the plurality of polynucleotides have members that encode in the primer binding sequences of a specific amino acid residue difference from a defined plurality of amino acid residue differences relative to a reference amino acid sequence such that the plurality of polynucleotide fragments encode all of a selected plurality of amino acid residues differences from the defined plurality of amino acid residue differences; and wherein the plurality of polynucleotide fragment comprises members for assembling two or more different amino acid sequence permutations of the defined amino acid differences. In some embodiments, the plurality of polynucleotide fragments comprises members sufficient for assembling all of the possible amino acid sequence permutations of the selected plurality of amino acid residue differences. In some embodiments, the members of the plurality are amplicons formed using the forward and reverse primers.

As will be apparent to the skilled the artisan, the methods described herein can be practiced using standard techniques available to the skilled artisan, such as that described in Sambrook et al., 2001, Molecular Cloning: A Laboratory Manual, 3rd Ed., Cold Spring Harbor Laboratory Press, NY and Ausubel et al., 1989, Current Protocols in Molecular Biology, Greene Publishing Associates and Wiley Interscience, N.Y (updates up to 2008). Oligonucleotides can be synthesized using known chemical methodologies, such as those based on phosphoramidite solid phase synthesis methodologies (See, e.g., Wright, et al., 1993, Tetrahedron Letters 34, 3373-3376; Caruthers, 1991, Acc. Chem. Res. 24, 278-284; and references cited herein).

Also disclosed herein are computer implemented systems in the form of computer software for carrying out the methods described above. In some embodiments, the computer program product comprises a machine readable storage medium having program instructions comprising codes for each of the steps of: (a) importing a reference sequence and a list of mutations associated with the sequence, (b) creating list of permutations based on the list of mutations, (c) selecting a defined permutation of the amino acid sequence, (d) identifying overlapping polynucleotide fragments from a library of amplicons (e.g., as prepared in **FIG. 5**), (e) determining the number of variants and if the number of variants is less than the total number of desired variants, reiterating steps (a) to (d).

In some embodiments, the computer program product comprises a machine readable storage medium having program instructions comprising codes for each of the steps of: (a) selecting a variant (an amino acid sequence permutation) and generating its corresponding polynucleotide sequence based on a reference sequence, (b) creating a forward oligonucleotide primer for a fragment with a first mutation, (c) scanning the sequence from the first mutation to the next mutation or to the end of the gene and creating a reverse oligonucleotide primer for the next mutation, (d) and if the next mutation is proximate to the first mutation, placing the next mutation on the same forward oligonucleotide, (e) reiterating steps (b) to (d) until ends of polynucleotide variant n is reached.

As shown in the illustrations of **FIG. 4****,** **FIG. 5****,** and **FIG. 6****,** the computer implemented programs for selection of the amplicons, selection of oligonucleotide primers, and storage in addressable formats can be integrated to permit automation of various steps of the methods of the disclosure.

As described herein, in some embodiments, the method can be used to synthesize polynucleotides encoding polypeptides having a defined set of mutations selected from a plurality of defined differences in amino acid residues from a reference sequence. The methods herein allow efficient synthesis of various permutations of amino acid sequences based on the amino acid residue differences. Efficient synthesis of polynucleotides encoding various amino acid sequence permutations is useful for a variety protein engineering applications. See, e.g., US application publication US20060195947; US application publication. US20050153417; and US Patent No. 7,220,566. In some embodiments, the methods can be use to synthesize polynucleotides encoding enzyme variants having improved properties based on a set of mutations known to affect different properties of the enzyme. For example, some mutations can affect, among others, enzyme activity, thermal stability, substrate specificity, stereoselectivity, stereospecificity, and refractoriness to product inhibition. While traditional techniques of random mutagenesis and protein evolution techniques can lead to identification of mutations affecting these various enzyme properties, many of these mutations can occur independently of the others. Using the methods herein, various permutations of mutations affecting different traits, such as enzyme activity, substrate specificity, and thermal stability can be made and screened to identify engineered enzymes having desired multiple altered traits.

The methods provided herein provide surprising efficiency and accuracy in generating large libraries of polynucleotide variants comprising various permutations of sequence changes. For example, the protein sequence for the gulonolactone (L-) oxidase (GLO) from *Rattus norvegicus* (accession: gi-92090602-sp-P10867.3-GGLO_RAT) can be back-translated to provide a 1.3 kb DNA sequence that can be used as a template to design 90 polynucleotide variants, each encoding a variant polypeptide having a different combination of from three to five amino acid substitutions. For example, a list of 90 permutations of from 3 to 5 amino acid substitutions can be selected from the following list of 10 possible substitutions: T28S, D95A, S156N, G175S, R212D, I251E, F302S, H330I, Y370G, and K423N. The 90 different permutations of amino acid substitutions encoded by the polynucleotide variants were as follows: D95A/F302S/H330I/K423N; D95A/F302S/Y370G; D95A/G175S/H330I; D95A/G175S/H330I/Y370G/K423N; D95A/G175S/R212D/F302S/Y370G; D95A/G175S/R212D/H330I; D95A/G175S/R212D/Y370G/K423N; D95A/I251E/F302S/K423N; D95A/I251E/H330I; D95A/I251E/K423N; D95A/I251E/Y370G; D95A/R212D/F302S; D95A/R212D/I251E/F302S; D95A/S156N/F302S/H330I/K423N; D95A/S156N/G175S; D95A/S156N/G175S/H330I/Y370G; D95A/S156N/G175S/I251E/F302S; D95A/S156N/I251E/H330I; D95A/S156N/I251E/K423N; D95A/S156N/K423N; D95A/S156N/R212D/I251E; F302S/H330I/K423N; G175S/F302S/Y370G/K423N; G175S/H330I/K423N; G175S/I251E/F302S; G175S/R212D/H330I; G175S/R212D/I251E/H330I; G175S/R212D/K423N; G175S/R212D/Y370G; G175S/R212D/Y370G/K423N; H330I/Y370G/K423N; I251E/H330I/Y370G; I251E/H330I/Y370G; I251E/Y370G/K423N; R212D/F302S/Y370G/K423N; R212D/H330I/K423N; R212D/I251E/F302S; R212D/I251E/F302S/H330I; R212D/I251E/Y370G; R212D/I251E/Y370G; S156N/F302S/H330I; S156N/F302S/K423N; S156N/F302S/Y370G; S156N/G175S/F302S/Y370G; S156N/G175S/I251E/F302S; S156N/G175S/K423N; S156N/G175S/K423N; S156N/G175S/R212D/F302S/H330I; S156N/I251E/F302S/H330I; S156N/I251E/H330I/Y370G; S156N/I251E/H330I/Y370G/K423N; S156N/I251E/Y370G; S156N/R212D/F302S/H330I/Y370G; S156N/R212D/K423N; T28S/D95A/G175S/F302S; T28S/D95A/G175S/F302S/Y370G; T28S/D95A/H330I; T28S/D95A/I251E; T28S/D95A/I251E/F302S/K423N; T28S/D95A/R212D; T28S/D95A/S156N/H330I/Y370G; T28S/D95A/S156N/R212D; T28S/D95A/S156N/R212D; T28S/D95A/S156N/R212D/Y370G; T28S/D95A/Y370G; T28S/D95A/Y370G/K423N; T28S/F302S/K423N; T28S/G175S/H330I; T28S/G175S/H330I/Y370G; T28S/G175S/I251E/F302S; T28S/G175S/I251E/F302S/Y370G; T28S/G175S/I251E/H330I; T28S/G175S/I251E/K423N; T28S/H330I/K423N; T28S/I251E/F302S/H330I/K423N; T28S/R212D/F302S/H330I; T28S/R212D/H330I; T28S/R212D/I251E/F302S; T28S/R212D/I251E/Y370G/K423N; T28S/R212D/Y370G; T28S/S156N/F302S/H330I/Y370G; T28S/S156N/F302S/Y370G; T28S/S156N/F302S/Y370G; T28S/S156N/G175S; T28S/S156N/G175S; T28S/S156N/G175S/I251E; T28S/S156N/G175S/I251E/K423N; T28S/S156N/R212D/I251E/H330I; T28S/S156N/R212D/I251E/K423N; and T28S/S156N/R212D/K423N.

Software (e.g., as described in Figures 5-7) can be used to determine a total of only 55 amplicons, corresponding to polynucleotide variant fragments with overlapping regions of sequence, can be used to assemble the 90 polynucleotide variants in a Round 2 SOE-PCR reaction. Software also can be used to determine that a total of only 22 oligonucleotide primers are needed in 55 separate Round 1 PCR reactions with the 1.3 kb reference polynucleotide as template to generate the necessary 55 amplicons. The 22 oligonucleotide primers are only 30 or 33 nucleotides in length, and include mutagenic primers comprising nucleotide changes in the middle of the sequence (e.g., at nucleotides 15-17).

Thus, in accordance with the methods disclosed herein, construction of the 90 different polynucleotide variants requires synthesis of only 22 relatively short oligonucleotides (30-mer to 33-mers), a first Round 1 PCR reaction to create the 55 amplicons (*i.e.,* polynucleotide variant fragments), and a second Round 2 SOE-PCR reaction in which the 55 amplicons are pooled in various combinations (with forward and reverse flanking primers) to allow SOE-PCR assembly of the 90 polynucleotide variants. In preparing the Round 2 SOE-PCR reactions, each of the 55 amplicons can be reused on average 7.8 times, with certain fragments used only once or twice, and others used as many as 36 times.

The workflow of the Round 1 and Round 2 reactions can be controlled by software-generated worklists (e.g., as in Figs. 4 and 6) which are used to run the Tecan robotics for the liquid handling. The worklists for this illustrative 90 variant library construction call for only 110 liquid handling operations for the Round 1 PCR reactions to generate the 55 amplicons using 22 primers, and only 430 liquid handling operations for the Round 2 SOE-PCR assembly reactions to prepare the 90 full length polynucleotide variants from the 55 amplicons.

The accuracy of the polynucleotide variant sequences provided by the methods disclosed herein can be determined by further steps of cloning and sequencing each of the plurality of constructs from the Round 2 reactions. As illustrated by the Examples (below), the methods disclosed herein result in surprisingly high level of correct sequences (full-length perfect (FLP) sequences) - *i.e.,* sequences which have the desired nucleotide changes relative to the reference polynucleotide.

At least some of the surprising advantages of the methods disclosed herein are in the greater accuracy of the large polynucleotide variant libraries produced. In some embodiments, the methods can be used to prepare an addressable library of at least 10 different polynucleotide variants, each comprising at least one defined sequence difference relative to a reference polynucleotide sequence, wherein at least an average of 75% of the polynucleotide variant sequences are correct sequences (e.g., the sequences comprising the full length reference sequence with the defined nucleotide differences introduced by the primers used in the method). In some embodiments, the methods provide an addressable library of at least 10, 20, 30, 40, 50, 60, 70, 80, 90, 100, 150, 200 or more different polynucleotide variants, each comprising at least one defined sequence difference relative to a reference polynucleotide sequence, wherein at least 75%, 80%, 85%, 90%, 95% or greater of the polynucleotide variant sequences are correct - e.g., FLP by sequence analysis.

In certain embodiments, the methods disclosed herein comprising a plurality of Round 1 PCR reactions using a reference polynucleotide template and a plurality of Round 2 SOE-PCR amplicon assembly reactions can be used to prepare an addressable library of 10 or more polynucleotide variants of reference polynucleotide of at least 500 bp, 750 bp, 1000 bp, 1250 bp, 1500 bp or more, each variant comprising from about 1-30, 1-25, 1-20, 1-15, 3-30, 3-20, or 3-15 nucleotide changes relative to the reference polynucleotide, wherein the Round 1 PCR reactions comprise about 6-300, 6-200, 6-100, 6-50, 6-40, 6-30, 6-25, 6-20, 6-15, or as few as 6-10 different oligonucleotide primers, and at least 75%, 80%, 85%, 90%, 95% or greater of the polynucleotide variant sequences are full-length perfect.

In some embodiments, various permutations of polynucleotide fragments (e.g., selected from an addressable library) can be assembled into an addressable library of polynucleotide variants each encoding a different variant polypeptide having a defined amino acid residue difference. Each of these polynucleotide variants can then be cloned into an expression system to generate an addressable library of clones, each capable of generating a different variant polypeptide. This addressable library of clones can be transformed into cells (*e.g., E. coli*), for translation, and automated plating and picking of colonies (*i.e.,* viable transformants). Sequencing then can be carried out to confirm the combination of mutations in each variant polypeptide sequence so generated. Assay (e.g., via high-throughput screening) of the variant polypeptides for desired altered traits can be carried out on all of the variant polypeptides, or optionally on only those variant polypeptides confirmed by sequencing as having the desired combination of mutations.

Alternatively, the addressable library of polynucleotide variant each encoding a different variant polypeptide having a defined amino acid residue difference can be combined (e.g., pooled) and cloned into an expression system, thereby creating a pooled library of clones. This pooled library of clones can be transformed (*e.g.,* in a single transformation step) into cells for translation, plating, and picking of colonies (*i.e.,* viable transformants). Assay of colonies from this pooled library of clones can be carried out (*e.g.,* via high-throughput screening) before sequencing to identify polynucleotide variants encoding polypeptides having desired altered traits. Once such "hit" for an altered trait is identified, it can be sequenced to determine the specific combination of mutations present in the polynucleotide variant sequence. Optionally, those variants encoding polypeptides not having the desired altered traits sought in assay need not be sequenced. Accordingly, the pooled library of clones method can provide more efficiency by requiring only a single transformation rather than a set of parallel transformation reactions.

Analogously, the method can also be used to generate various permutations of mutation combinations to examine the structural features of biologically important proteins. For example, receptors involved in signal transduction of extracellular molecules act via interaction with other receptors as well as various intracellular proteins. These complex interactions can affect different cellular signaling processes from the same type of receptor molecule. Both negative and positive signaling can be initiated by the same receptor. A specific example is G-coupled protein receptors, which interact with βγ, Gsα, and Giα proteins. See, e.g., Morris et al., 1999, Physiol. Rev. 79:1373-1430. Because mutations at different domains of the receptor can have different effects, the methods herein provide an efficient process for generating different permutations of mutation combinations known to affect different aspects of receptor function, thereby permitting studies on the structural and associated biological functions of the protein of interest.

While the methods for generating different permutations of a polynucleotide sequence have been illustrated for generating polynucleotides encoding various permutations of a polypeptide from a defined set of amino acid residue differences, it is to be understood that the methods can be adapted generally for generating permutations of polynucleotide sequences. For example, the methods herein can be used to generate different permutations of functional polynucleotides, such as genes for ribosomal RNAs. Various rRNAs form nucleoprotein complexes that participate in protein synthesis in prokaryotes and eukaryotes. Many antibiotics function by disrupting ribosome function and are known to interact with defined regions of certain rRNAs. Various mutations have been identified that affect protein synthesis and these regions are correlated with sites of interaction with antibiotics. See, e.g., Yassin et al., 2005, Proc Natl Acad Sci. USA 102(46):16620-16625.

Using the methods described herein, various permutations of known mutations affecting ribosomal RNA function can be synthesized and the effects of certain combination of mutation examined. Other applications will be apparent to the skilled artisan.

### 5. EXAMPLES

### Example 1: Preparation of Amplicons

Oligonucleotide Preparation. Oligonucleotide primers at 200uM concentration are diluted to 4uM with sterile water in an Axygen HalfDeep 96 (1.1mL) plate. For most positions on the microtiter plate, adding 10 µL of oligo to 490 µL dH₂O will be sufficient. For positions A01 and D01 on the oligonucleotide plate, the common forward and reverse primers, a larger volume may be required. The maximum aspiration volumes in the output report in the aspirate and dispense volumes section is verified prior to the next step.

Round 1 - Formation of Amplicons by PCR. Tecan robotics are used to aliquot 5 µL of each forward and reverse oligonucleotide primer into BioRad HardShellPCR96 plates (Tecan script output) and to add 40 µL of master mix. Perform Round 1 PCR and verify amplification using a 2% 96 well e-gel. Reagent for the PCR is as follows: 5 µL 10x Herculase buffer, 1 µL 40 mM dNTPs, 1 µL of 100 ng/µL of SOE template, 2.5 units Herculase polymerase (Stratagene, LaJolla, CA, USA). PCR is carried out as follows: 2 min denaturation at 95°C followed by cycling at 95°C for 30s, 56°C for 30s, 72°C for 1min/Kb. The number of cycles is 17.

Treatment with ExoSAP-it. For Round 1 of PCR, 25 µL of the reaction product is transferred to a fresh 96 well plate, and 2 µL ExoSAP-It (USB Corp., Ohio, USA) plus 0.5 µL Dpnl is added and cycling performed (manual transfer 37°C 1 hour 80°C 15 min). Samples are diluted to a final volume of 100 µL by the addition of 73 µL dH₂O and pooled into another BioRad HardShellPCR96 PCR plate using a Tecan script.

### Example 2: Assembly of Amplicons and Analysis of Products

Round 2 - Assembly and SOE-PCR Tecan robotics are used to aliquot a 15 µL pool of fragments (i.e., Round 1 amplicons) into BioRad HardShellPCR96 plates (Tecan script output) and to add 35 µL of master mix (5 µL of 10X Herculase Buffer, 1 µL of 40 mM dNTPs, 0.2 µL of forward primers, 0.2 µL of reverse primers, 2.5 units Herculase enzyme, and 28.1 µL dH₂O. Perform PCR and verify amplification using a 2% 96 well e-gel. PCR is carried out as follows: 2 min denaturation at 95°C following by cycling at 95°C for 30s, 56°C for 30s, 72°C for 1min/Kb. The number of cycles is 17.

96-well plate purification. Purify all samples using Zymo ZR-96 PCR clean up 96 well plates (manufacturers protocol with the following modifications) (Zymo Research, CA, USA). Perform all centrifuge steps at 2800 rpm for 10 minutes. To elute the DNA, apply 25 µL dH₂O of temperature 55°C directly to the silica membrane, spin for 10 minutes and repeat. Using this method 48-50 µL of product is recovered.

Restriction enzyme Bgll digestion. Transfer 30 µL of each purified insert is to a fresh semi-skirted PCR plate, add 20 µL of Bgll digestion master mix (5 µL 10X NEB Buffer 3, 20 units Bgll (New England Biolabs, MA, USA), 13uL dH₂O) to all samples and incubate at 37°C for 4 hrs. Bgl digestion is for cloning into Bgll site of an expression vector.

96-well plate purification. Purify all samples using Zymo ZR-96 PCR clean up 96-well plates (manufacturers protocol with the following modifications). Perform all centrifuge steps at 2800 rpm for 10 minutes. To elute the DNA, apply 25 µL dH₂O at temperature 55°C directly to the silica membrane, spin for 10 min, and repeat. Using this method, 48-50 µL of product is recovered. Verify product recovery using a 2% 96 well e-gel.

Ligations to expression vector. Transfer 3 µL of each purified insert to a fresh plate and add 27uL of ligation master mix to the samples. Incubated for 14 hours at 16°C followed by 15 minutes at 65°C, followed by 8°C hold. Ligation mix: 3 µL 10X Ligase Buffer (New England Biolabs, MA, USA), 1 µL Bgll digested vector (50 ng/µL), 400 units T4 Ligase (New England Biolabs, MA, USA), 22 µL dH₂O.

HTP-Transformation. Transfer 2uL of each ligation reaction to 20uL of TSS chemically competent cells and incubate on ice in a metal block for at least 15 min. Heat shock at 42°C for 35 seconds and return to the metal block for 2 min. Add 80 µL of 37°C SOC media to each sample. Incubate at 37°C for 1 hour before plating.

Plating. Plate the transformation mixture of 48-well divided Q-trays using the Tecan. Dispense three 5 mm bead to each well of the Q-tray using a bead dispenser. Use the Tecan to dispense 40 µL per well of transformation mixture. Grow the transformants overnight at 37°C.

Picking and culturing. For sequence verified plates, request that two colonies per Q-tray well be picked to two separate Nunc flat bottom plates containing LB, CAM, and 1% glucose. For non-sequence verified variant plates, request that three colonies per Q-tray well be picked to three separate Nunc flat bottom plates containing LB, CAM, and 1% glucose.

Colony PCR To perform colony PCR on one of the two replicate master plates, add 2uL of culture to the standard colony PCR master mix and perform colony PCR. ExoSAP-it is used to clean up the PCT product as follows: Transfer 5 µL of PCR sample to a new PCR plate containing 2 µL ExoSAP-it. Incubate at 37°C for 15 min and 80°C for 15 min. Dilute the samples to a final volume of 40 µL by adding 33 µL of dH₂O.

Sequencing of PCR products. Add 4 µL of ImM sequencing primer to the sequencing plate. Add 4 µL of cleaned up PCR sample.

### Example 3: Generation of a set of 190 different polynucleotide variants each encoding a polypeptide having a single amino acid change relative to a reference polypeptide

Experimental design: A reference polynucleotide of 1359 bp (encoding an enzyme of453 amino acids) was selected. A total of 190 amino acid residue differences from the reference sequence were selected based upon sequence changes seen in homologous enzymes. The 190 variants were made as individual polynucleotides encoding the 190 different proteins to be expressed and tested. Each of the 190 polynucleotide variants was assembled by combining two amplicons comprising the desired single codon change in their overlap region (as prepared in Round 1 below) in a SOE reaction (Round 2 below).

Oligonucleotide preparation: A total of 382 oligonucleotide primers for PCR were designed and synthesized according to standard methods. Oligonucleotides were generally 31 nucleotides (nt) in length with the desired change(s) to the codon of interest located in the middle (∼ base 15) of the oligonucleotide primer. All oligonucleotides were diluted to 4 µM with sterile water in an Axygen HalfDeep 96 (1.1 mL) plate.

Round 1 - Formation of Amplicons by PCR: Each amplicon, corresponding to a polynucleotide variant fragment, was generated in a PCR reaction using a vector comprising 1359 nt reference polynucleotide as template and using a mutagenic primer in combination with a common flanking primer (annealing to the vector upstream or downstream of the gene containing no mutation). Reaction workflow, conditions and clean-up were as described in Example 1.

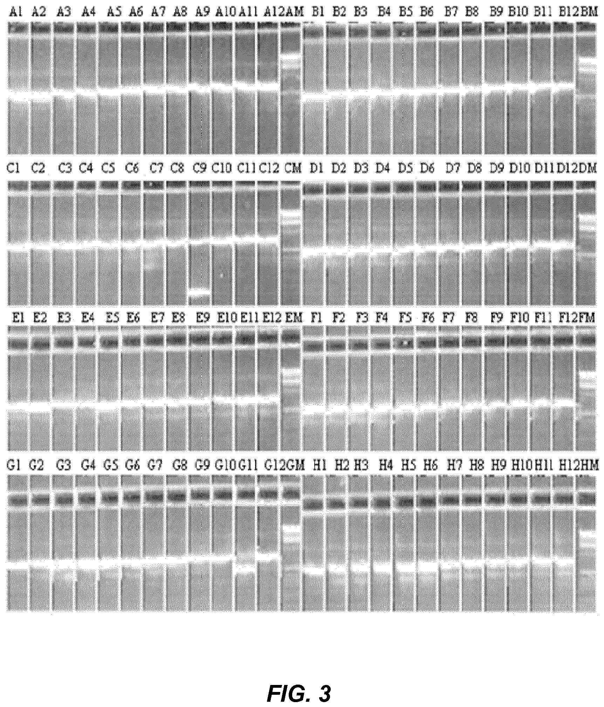

Round 2 - Assembly of Amplicons. Purified amplicons from Round 1 were pooled such that 2 amplicons with overlapping regions comprising the desired codon sequence change for each of the 190 polynucleotide variants and each pool was aliquoted into the well of a 96-well plate as described in Examples 1 and 2. Common flanking forward and reverse primers were added to each pool and PCR was carried out as described in Example 2 resulting in assembly of the amplicons (i.e., polynucleotide fragments) to form the full length polynucleotide variant. Inspection of the assembly reactions was carried out on an agarose gel and were found to contain product of the expected size. (*See e.g.,* Fig. 3.)

Sequence Analysis of Polynucleotide Variants. After purification using Xymo ZR-96 PCR clean up, products were cloned into an expression vector, and each ligation was transformed into E. *coli* host cells. Two colonies from each transformation were picked and plasmid DNA was prepared for DNA sequencing. One sample from each transformation was sequenced using sequencing primers internal to and flanking the gene. Of the 190 polynucleotide variants, 160 (84%) were shown to have only the full length perfect (FLP) sequence with the desired codon sequence changes. By sequencing the second plasmid preparation of the 30 variants that were incorrect, 25 additional correct sequences were identified. This brought the overall success rate to 97% correct sequences. (185 of the desired 190 polynucleotides were identified.) The polynucleotides were expressed and the variant polypeptides assayed.

### Example 4: Generation of a set of 96 different polynucleotide variants each encoding a polypeptide having a three amino acid changes relative to a reference polypeptide.

Experimental Design: A reference polynucleotide of 1359 nt was selected. 96 variants were designed, each containing three mutations relative to the reference sequence. The 96 variants were made as individual polynucleotides each encoding one of 96 different proteins to be expressed and tested. Each of the 96 polynucleotide variants was assembled by combining four amplicons comprising the desired codon changes in their overlap region (as prepared in Round 1 below) in a SOE reaction (Round 2 below).

Oligonucleotide Preparation: A total of 130 oligonucleotide primers were designed and synthesized according to standard methods. Oligonucleotides were generally 31 nt in length with the desired change(s) to the codon of interest in the middle (∼ base 15) of the oligonucleotide. All oligonucleotides were diluted to 4µM with sterile water in an Axygen HalfDeep 96 (1.1ml) plate.

Round 1 - Formation of Amplicons by PCR: Each amplicon, corresponding to a polynucleotide variant fragment, was generated in a PCR reaction using a vector comprising 1359 nt reference polynucleotide as template and using a mutagenic primer in combination with another mutagenic primer or a common flanking primer (annealing to the vector upstream or downstream of the gene containing no mutation). Reaction workflow, conditions and clean-up were as described in Example 1.

Round 2 - Assembly of Amplicons. Purified amplicons from Round 1 were pooled such that 4 amplicons with overlapping regions comprising the desired codon sequence changes for each of the 96 polynucleotide variants and each pool was aliquoted into the well of a 96-well plate as described in Examples 1 and 2. Common flanking forward and reverse primers were added to each pool and PCR was carried out as described in Example 2 resulting in assembly of the amplicons (i.e., polynucleotide fragments) to form the full length polynucleotide variant. Inspection of the assembly reactions was carried out on an agarose gel and were found to contain product of the expected size. (*See e.g.,* Fig. 3.)

Sequence analysis of Polynucleotide product. After purification using Xymo ZR-96 PCR clean up, products were cloned into an expression vector, and each ligation was transformed into E. coli host cells. Two colonies from each transformation were picked and plasmid DNA was prepared for DNA sequencing. One sample from each transformation was sequenced using sequencing primers internal to and flanking the gene. Of the 96 polynucleotide variants, 82 (85%) were determined to have only the correct FLP sequence with the desired changes.

### Example 5: Generation of a set of 96 different polynucleotide variants each encoding a polypeptide having from one to six amino acid changes relative to a reference polypeptide.

Experimental design: A reference polynucleotide of 1056 nt was selected. 96 variants were designed, each containing from one (1) to six (6) mutations from the reference sequence. The 96 variants were made as individual polynucleotides each encoding one of the 96 different proteins to be expressed and tested. Each of the 96 polynucleotide variants was assembled by combining from two (e.g., for variant encoding a single amino acid change) to seven (e.g., for variant encoding six amino acid changes) amplicons comprising the desired codon changes in their overlap region (as prepared in Round 1 below) in a SOE reaction (Round 2 below).

Oligonucleotide preparation: A total of 108 oligonucleotide primers were designed and synthesized according to standard methods. Oligonucleotides were generally 31 nt in length with the desired change(s) to the codon of interest in the middle (∼ base 15) of the oligonucleotide. If two amino acid changes were close together, a longer oligo was designed to encode for both changes to be incorporated. All oligonucleotides were diluted to 4µM with sterile water in an Axygen HalfDeep 96 (1.1 mL) plate.

Round 1 - Formation of Amplicons by PCR: Each amplicon, corresponding to a polynucleotide variant fragment, was generated in a PCR reaction using a vector comprising 1056 nt reference polynucleotide as template and using a mutagenic primer in combination with another mutagenic primer or a common flanking primer (annealing to the vector upstream or downstream of the gene containing no mutation). Reaction workflow, conditions and clean-up were as described in Example 1.

Round 2 - Assembly of Amplicons. Purified pooled amplicons (from 2 to7 amplicons/polynucleotide) were aliquoted into plates as described in Example 1. Common flanking forward and reverse primers were added and PCR was carried out as described in Example 1.

Sequence Analysis of Polynucleotide Product. After purification using Xymo ZR-96 PCR clean up, products were cloned into an expression vector, and each ligation was transformed into E. *coli* host cells. Two colonies from each transformation were picked and plasmid DNA was prepared for DNA sequencing. One sample from each transformation was sequenced using sequencing primers internal to and flanking the gene. As shown in Table 1 (below), of the 96 polynucleotide variants, 72 (75%) were shown to have only the correct FLP sequence with the desired 2-7 codon changes.

**Table 1: 96 Variants Constructed - 88 Variants Required to Make Final Plate**

| **Sequencing Set** | **1 (n=96)** | **2 (n=96)** | **3 (n=16)** |
|---|---|---|---|
| Total Correct | 72 | 84 | 92 |
| Failed Sequences | 5 | 3 | 2 |
| Cross Contamination | 5 | 2 | 0 |
| Coding Mutations | 8 | 2 | 0 |
| Indels | 6 | 4 | 2 |

## Claims

1. A method of synthesizing a plurality of polynucleotide variants each having at least one defined nucleotide difference relative to a reference polynucleotide sequence, the method comprising:
(a) separately amplifying a reference polynucleotide template with each of a plurality of pairs of forward and reverse primers, wherein the plurality of pairs of forward and reverse primers comprises a plurality of defined nucleotide differences and wherein each pair generates an amplicon comprising a sequence capable of binding to an adjacent overlapping sequence of at least one other amplicon, thereby generating an addressable library of amplicons each corresponding to a segment of the reference polynucleotide sequence having a defined nucleotide difference;
(b) separately assembling a plurality of sets of amplicons from the addressable library of amplicons, wherein each set comprises amplicons having adjacent overlapping sequences capable of binding to form the full length of the reference polynucleotide sequence;
(c) replicating the plurality of sets of assembled amplicons, thereby synthesizing a plurality of polynucleotide variants each having at least one defined nucleotide difference relative to the reference polynucleotide,
wherein the reference polynucleotide encodes a reference polypeptide and each of the plurality of polynucleotide variants encodes a polypeptide having at least one defined amino acid sequence difference as compared to the reference polypeptide,
and wherein the method is used to synthesize a polynucleotide variant encoding a polypeptide with an improvement in a desired property of a reference polypeptide encoded by the reference polynucleotide.

2. The method of claim 1, wherein the method further comprises the step of cloning each of the plurality of polynucleotide variants into an expression vector, and wherein the method optionally further comprises transforming cells with the expression vectors.

3. The method of claim 2, wherein the method further comprises screening the transformed cells for activity of the polypeptides encoded by the polynucleotide variants, and optionally further comprises isolating at least one polypeptide encoded by the polynucleotide variants.

4. The method of any preceding claim, wherein the defined amino acid sequence differences in the polypeptides encoded by the polynucleotide variants are mutations identified as associated with changes in a desired property of the reference polypeptide.

5. The method of any preceding claim, wherein the method is used:
(i) to synthesize polynucleotides encoding enzyme variants having an improved property based on a set of mutations known to affect a property of the enzyme, and optionally to synthesize polynucleotides encoding enzyme variants having improved properties based on a set of mutations known to affect different properties of the enzyme; or
(ii) to synthesize polynucleotides encoding polypeptides with different permutations of mutation combinations known to affect different aspects of receptor function.

6. The method of claim 1, wherein:
(i) the plurality of polynucleotide variants comprises at least 10, at least 25, at least 35, at least 50, at least 75, at least 90, at least 120, at least 150, or at least 180, different polynucleotide variants;
(ii) each of the plurality of polynucleotide variants comprises at least 2, at least 3, at least 6, at least 9, at least 12, at least 15, at least 18, at least 21, at least 24, at least 27, or at least 30, defined nucleotide differences relative to the reference polynucleotide sequence;
(iii) at least one of the plurality of polynucleotide variants comprises at least 2, at least 3, at least 6, at least 9, at least 12, at least 15, at least 18, at least 21, at least 24, at least 27, or at least 30, defined nucleotide differences relative to the reference polynucleotide sequence;
(iv) at least one of the plurality of sets of amplicons comprises at least 3, at least 5, at least 7, or at least 10, different amplicons;
(v) at least one of the plurality of sets of amplicons comprises a bridging polynucleotide comprising no nucleotide difference relative to the reference polynucleotide sequence;
(vi) the lengths of the forward and reverse primers are from 20 to 50 nucleotides, from 20 to 40 nucleotides, or from 25 to 35 nucleotides;
(vii) at least 65%, at least 75%, at least 85%, or at least 95% of the synthesized plurality of polynucleotide variants comprise the full length reference sequence with the defined nucleotide differences introduced by the primers used in the method;
(viii) the length of the reference polynucleotide sequence is at least 500 bp, at least 750 bp, at least 1000 bp, at least 1250 bp, or at least 1500 bp;
(ix) the plurality of pairs of forward and reverse primers comprises from 6 to 200, from 6 to 150, from 6 to 100, from 6 to 50, from 6 to 40, from 6 to 30, from 6 to 25, from 6 to 20, or from 6 to 15, different oligonucleotides; and/or
(x) the plurality of pairs of forward and reverse primers comprises 400 or fewer, 300 or fewer, 200 or fewer, 100 or fewer, 50 or fewer, or 25 or fewer different oligonucleotides.

7. The method of claim 1, wherein primers that anneal to primer binding sequences that flank the polynucleotide variants are added to amplify the polynucleotide variants in additional amplification reactions, and wherein optionally:
(i) the flanking primers incorporate recognition sequences for restriction enzymes; and/or
(ii) the flanking primers comprise sequences that allow for direct *in vitro* expression using a coupled transcription-translation system for synthesis of the protein product without the need for transformation into a host organism.

8. An addressable library of polynucleotide variants comprising a plurality of polynucleotide variants synthesized according to the method of claim 1, each having at least one defined nucleotide difference relative to a reference polynucleotide sequence, wherein the library comprises at least 10 polynucleotide variants, wherein at least one of the polynucleotide variants comprises at least two defined nucleotide differences relative to the reference polynucleotide sequence, wherein the reference polynucleotide encodes a reference polypeptide and each of the plurality of polynucleotide variants encodes a polypeptide having at least one defined amino acid sequence difference as compared to the reference polypeptide, and wherein the library comprises a polynucleotide variant encoding a polypeptide with an improvement in a desired property of a reference polypeptide encoded by the reference polynucleotide.

9. The addressable library of polynucleotide variants according to claim 8, wherein the library comprises at least 25, at least 35, at least 50, at least 75, at least 90, at least 120, at least 150, or at least 180, different polynucleotide variants.

10. An addressable library of amplicons, wherein each member of the library of amplicons comprises at least one defined nucleotide difference relative to a reference polynucleotide sequence and an overlapping adjacent region capable of binding to the overlapping adjacent region of at least one other amplicon in the library, wherein the library comprises at least 5 different amplicons, wherein the library of amplicons comprises members for assembling two or more different full length polynucleotide variants comprising defined nucleotide differences relative to the reference polynucleotide sequence, wherein the reference polynucleotide encodes a reference polypeptide and each of the plurality of polynucleotide variants encodes a polypeptide having at least one defined amino acid sequence difference as compared to the reference polypeptide, and wherein the library of amplicons comprises at least one set of amplicons capable of binding to form the full length of the reference polynucleotide sequence and encoding a polypeptide with an improvement in a desired property of a reference polypeptide encoded by the reference polynucleotide.

11. The addressable library of amplicons according to claim 10, wherein the library comprises at least 10, at least 20, at least 30, at least 40, at least 50, at least 75, or at least 100, different amplicons.

12. The addressable library of claim 10, wherein the reference polynucleotide sequence encodes a reference polypeptide and the plurality of amplicons comprises members sufficient for assembling all of the possible nucleotide differences encoding a selected plurality of amino acid residue differences.

13. An addressable library of clones, each clone capable of generating a different variant polypeptide having at least one defined amino acid sequence difference as compared to a reference polypeptide, generated by cloning the addressable library of claim 8 into an expression system.

14. An addressable library of transformed cells, each cell capable of generating a different variant polypeptide having at least one defined amino acid sequence difference as compared to the reference polypeptide, generated by transforming the addressable library of clones of claim 13 into cells.

15. A robotic system comprising an addressable library of polynucleotide variants according to claim 8, an addressable library of amplicons according to claim 10 and/or an addressable library of transformed cells according to claim 14.

## Patentansprüche

1. Verfahren zur Synthese mehrerer Polynukleotidvarianten mit jeweils wenigstens einem definierten Nukleotidunterschied relativ zu einer Referenzpolynukleotidsequenz, wobei das Verfahren Folgendes umfasst:
(a) getrenntes Amplifizieren einer Referenzpolynukleotidmatrize mit jedem von mehreren Paar Vorwärts- und Rückwärtsprimern, wobei die mehreren Paar Vorwärts- und Rückwärtsprimer mehrere definierte Nukleotidunterschiede umfassen und wobei durch jedes Paar ein Amplifikat erzeugt wird, das eine Sequenz umfasst, die zur Bindung an eine benachbarte überlappende Sequenz wenigstens eines anderen Amplifikats fähig ist, wodurch eine adressierbare Bibliothek von Amplifikaten, die jeweils einem Segment der Referenzpolynukleotidsequenz mit einem definierten Nukleotidunterschied entsprechen, erzeugt wird;
(b) getrenntes Zusammensetzen mehrerer Sätze von Amplifikaten aus der adressierbaren Bibliothek von Amplifikaten, wobei jeder Satz Amplifikate mit benachbarten überlappenden Sequenzen umfasst, die zur Bindung unter Bildung der vollen Länge der Referenzpolynukleotidsequenz fähig sind;
(c) Replizieren der mehreren Sätze zusammengesetzter Amplifikate, wodurch mehrere Polynukleotidvarianten synthetisiert werden, die jeweils wenigstens einen definierten Nukleotidunterschied relativ zum Referenzpolynukleotid aufweisen,
wobei das Referenzpolynukleotid ein Referenzpolypeptid codiert und die mehreren Polynukleotidvarianten jeweils ein Polypeptid mit wenigstens einem definierten Aminosäuresequenzunterschied im Vergleich zum Referenzpolypeptid codieren und wobei das Verfahren zur Synthese einer Polynukleotidvariante verwendet wird, die ein Polypeptid mit einer Verbesserung bei einer gewünschten Eigenschaft eines vom Referenzpolynukleotid codierten Referenzpolypeptids codiert.

2. Verfahren nach Anspruch 1, wobei das Verfahren ferner den Schritt des Klonierens einer jeden der mehreren Polynukleotidvarianten in einen Expressionsvektor umfasst und wobei das Verfahren gegebenenfalls ferner Transformieren von Zellen mit den Expressionsvektoren umfasst.

3. Verfahren nach Anspruch 2, wobei das Verfahren ferner Screening der transformierten Zellen auf Aktivität der durch die Polynukleotidvarianten codierten Polypeptide und gegebenenfalls ferner Isolieren wenigstens eines durch die Polynukleotidvarianten codierten Polypeptids umfasst.

4. Verfahren nach einem vorhergehenden Anspruch, wobei es sich bei den definierten Aminosäuresequenzunterschieden bei den durch die Polynukleotidvarianten codierten Polypeptiden um als mit Änderungen einer gewünschten Eigenschaft des Referenzpolypeptids assoziiert identifizierte Mutationen handelt.

5. Verfahren nach einem vorhergehenden Anspruch, wobei das Verfahren verwendet wird, um:
(i) Polynukleotide, die Enzymvarianten mit einer verbesserten Eigenschaft codieren, basierend auf einem Satz bekannter Mutationen, die sich auf eine Eigenschaft des Enzyms auswirken, und gegebenenfalls Polynukleotide, die Enzymvarianten mit verbesserten Eigenschaften codieren, basierend auf einem Satz bekannter Mutationen, die sich auf unterschiedliche Eigenschaften des Enzyms auswirken, zu synthetisieren; oder
(ii) Polynukleotide, die Polypeptide mit unterschiedlichen Permutationen bekannter Mutationskombinationen, die sich auf unterschiedliche Aspekte von Rezeptorfunktion auswirken, zu synthetisieren.

6. Verfahren nach Anspruch 1, wobei:
(i) die mehreren Polynukleotidvarianten wenigstens 10, wenigstens 25, wenigstens 35, wenigstens 50, wenigstens 75, wenigstens 90, wenigstens 120, wenigstens 150 oder wenigstens 180 unterschiedliche Polynukleotidvarianten umfassen;
(ii) die mehreren Polynukleotidvarianten jeweils wenigstens 2, wenigstens 3, wenigstens 6, wenigstens 9, wenigstens 12, wenigstens 15, wenigstens 18, wenigstens 21, wenigstens 24, wenigstens 27 oder wenigstens 30 definierte Nukleotidunterschiede relativ zur Referenzpolynukleotidsequenz umfassen;
(iii) wenigstens eine der mehreren Polynukleotidvarianten wenigstens 2, wenigstens 3, wenigstens 6, wenigstens 9, wenigstens 12, wenigstens 15, wenigstens 18, wenigstens 21, wenigstens 24, wenigstens 27 oder wenigstens 30 definierte Nukleotidunterschiede relativ zur Referenzpolynukleotidsequenz umfasst;
(iv) wenigstens einer der mehreren Sätze von Amplifikaten wenigstens 3, wenigstens 5, wenigstens 7 oder wenigstens 10 unterschiedliche Amplifikate umfasst;
(v) wenigstens einer der mehreren Sätze von Amplifikaten ein Brückenpolynukleotid umfasst, das keinen Nukleotidunterschied relativ zur Referenzpolynukleotidsequenz umfasst;
(vi) die Vorwärts- und Rückwärtsprimer eine Länge von 20 bis 50 Nukleotiden, von 20 bis 40 Nukleotiden oder von 25 bis 35 Nukleotiden aufweisen;
(vii) wenigstens 65%, wenigstens 75%, wenigstens 85% oder wenigstens 95% der synthetisierten mehreren Polynukleotidvarianten die Volllängen-Referenzsequenz mit den durch die beim Verfahren verwendeten Primer eingeführten definierten Nukleotidunterschieden umfassen;
(viii) die Referenzpolynukleotidsequenz eine Länge von wenigstens 500 bp, wenigstens 750 bp, wenigstens 1000 bp, wenigstens 1250 bp oder wenigstens 1500 bp aufweist;
(ix) die mehreren Paar Vorwärts- und Rückwärtsprimer 6 bis 200, 6 bis 150, 6 bis 100, 6 bis 50, 6 bis 40, 6 bis 30, 6 bis 25, 6 bis 20 oder 6 bis 15 unterschiedliche Oligonukleotide umfassen; und/oder
(x) die mehreren Paar Vorwärts- und Rückwärtsprimer 400 oder weniger, 300 oder weniger, 200 oder weniger, 100 oder weniger, 50 oder weniger oder 25 oder weniger unterschiedliche Oligonukleotide umfassen.

7. Verfahren nach Anspruch 1, wobei Primer, die beim Annealing an Primerbindungssequenzen binden, die die Polynukleotidvarianten flankieren, zugegeben werden, so dass die Polynukleotidvarianten in zusätzlichen Amplifikationsreaktionen amplifiziert werden, und wobei gegebenenfalls:
(i) in die flankierenden Primer Erkennungssequenzen für Restriktionsenzyme eingebaut sind; und/oder
(ii) die flankierenden Primer Sequenzen umfassen, die eine direkte In-vitro-Expression unter Verwendung eines gekoppelten Transkription/Translation-Systems zur Synthese des Proteinprodukts ohne die Notwendigkeit einer Transformation in einen Wirtsorganismus ermöglichen.

8. Adressierbare Bibliothek von Polynukleotidvarianten, umfassend mehrere gemäß dem Verfahren nach Anspruch 1 synthetisierte Polynukleotidvarianten, die jeweils wenigstens einen definierten Nukleotidunterschied relativ zu einer Referenzpolynukleotidsequenz aufweisen, wobei die Bibliothek wenigstens 10 Polynukleotidvarianten umfasst, wobei wenigstens eine der Polynukleotidvarianten wenigstens zwei definierte Nukleotidunterschiede relativ zur Referenzpolynukleotidsequenz umfasst, wobei das Referenzpolynukleotid ein Referenzpolypeptid codiert und die mehreren Polynukleotidvarianten jeweils ein Polypeptid mit wenigstens einem definierten Aminosäuresequenzunterschied im Vergleich zum Referenzpolypeptid codieren und wobei die Bibliothek eine Polynukleotidvariante umfasst, die ein Polypeptid mit einer Verbesserung bei einer gewünschten Eigenschaft eines vom Referenzpolynukleotid codierten Referenzpolypeptids codiert.

9. Adressierbare Bibliothek von Polynukleotidvarianten gemäß Anspruch 8, wobei die Bibliothek wenigstens 25, wenigstens 35, wenigstens 50, wenigstens 75, wenigstens 90, wenigstens 120, wenigstens 150 oder wenigstens 180 unterschiedliche Polynukleotidvarianten umfasst.

10. Adressierbare Bibliothek von Amplifikaten, wobei jedes Mitglied der Bibliothek von Amplifikaten wenigstens einen definierten Nukleotidunterschied relativ zu einer Referenzpolynukleotidsequenz und einen überlappenden benachbarten Bereich mit der Fähigkeit zur Bindung an den überlappenden benachbarten Bereich wenigstens eines anderen Amplifikats in der Bibliothek umfasst, wobei die Bibliothek wenigstens 5 unterschiedliche Amplifikate umfasst, wobei die Bibliothek von Amplifikaten Mitglieder zum Zusammensetzen von zwei oder mehr unterschiedlichen Volllängen-Polynukleotidvarianten, die definierte Nukleotidunterschiede relativ zur Referenzpolynukleotidsequenz umfassen, umfasst, wobei das Referenzpolynukleotid ein Referenzpolypeptid codiert und die mehreren Polynukleotidvarianten jeweils ein Polypeptid mit wenigstens einem definierten Aminosäuresequenzunterschied im Vergleich zum Referenzpolypeptid codieren und wobei die Bibliothek von Amplifikaten wenigstens einen Satz von Amplifikaten, die zur Bindung unter Bildung der vollen Länge der Referenzpolynukleotidsequenz fähig sind und ein Polypeptid mit einer Verbesserung bei einer gewünschten Eigenschaft eines vom Referenzpolynukleotid codierten Referenzpolypeptids codieren.

11. Adressierbare Bibliothek von Amplifikaten gemäß Anspruch 10, wobei die Bibliothek wenigstens 10, wenigstens 20, wenigstens 30, wenigstens 40, wenigstens 50, wenigstens 75 oder wenigstens 100 unterschiedliche Amplifikate umfasst.

12. Adressierbare Bibliothek nach Anspruch 10, wobei die Referenzpolynukleotidsequenz ein Referenzpolypeptid codiert und die mehreren Amplifikate Mitglieder umfassen, die zum Zusammensetzen aller möglichen Nukleotidunterschiede, die ausgewählte mehrere Aminosäuresequenzunterschiede codieren, hinreichend sind.

13. Adressierbare Bibliothek von Klonen, die jeweils zur Erzeugung einer unterschiedlichen Polypeptidvariante mit wenigstens einem definierten Aminosäuresequenzunterschied im Vergleich zu einem Referenzpolypeptid fähig sind, erzeugt durch Klonieren der adressierbaren Bibliothek nach Anspruch 8 in ein Expressionssystem.

14. Adressierbare Bibliothek transformierter Zellen, die jeweils zur Erzeugung einer unterschiedlichen Polypeptidvariante mit wenigstens einem definierten Aminosäuresequenzunterschied im Vergleich zum Referenzpolypeptid fähig sind, erzeugt durch Transformieren der adressierbaren Bibliothek nach Anspruch 13 in Zellen.

15. Robotersystem, umfassend eine adressierbare Bibliothek von Polynukleotidvarianten gemäß Anspruch 8, eine adressierbare Bibliothek von Amplifikaten gemäß Anspruch 10 und/oder eine adressierbare Bibliothek transformierter Zellen gemäß Anspruch 14.

## Revendications

1. Procédé de synthèse d'une pluralité de variants de polynucléotide comportant chacun au moins une différence de nucléotide définie par rapport à une séquence de polynucléotide de référence, le procédé comprenant :
(a) l'amplification séparée d'une matrice de polynucléotide de référence avec chacune d'une pluralité de paires d'amorces sens et antisens, la pluralité de paires d'amorces sens et antisens comprenant une pluralité de différences de nucléotide définies et chaque paire générant un amplicon comprenant une séquence capable de se lier à une séquence chevauchante adjacente d'au moins un autre amplicon, de façon à générer une banque adressable d'amplicons correspondant chacun à un segment de la séquence de polynucléotide de référence ayant une différence de nucléotide définie ;
(b) assemblage séparé d'une pluralité d'ensembles d'amplicons de la banque adressable d'amplicons, chaque ensemble comprenant des amplicons ayant des séquences chevauchantes adjacentes capables de se lier pour former la pleine longueur de la séquence de polynucléotide de référence ;
(c) réplication de la pluralité d'ensembles d'amplicons assemblés, de façon à synthétiser une pluralité de variants de polynucléotide ayant chacun au moins une différence de nucléotide définie par rapport au polynucléotide de référence,
dans lequel le polynucléotide de référence code pour un polypeptide de référence et chacun de la pluralité de variants de polynucléotide code pour un polypeptide comportant au moins une différence de séquence d'acides aminés définie par rapport au polypeptide de référence, et dans lequel le procédé est utilisé pour synthétiser un variant de polynucléotide codant pour un polypeptide présentant une amélioration d'une propriété souhaitée d'un polypeptide de référence codé par le polynucléotide de référence.

2. Procédé selon la revendication 1, le procédé comprenant en outre l'étape de clonage de chacun de la pluralité de variants de polynucléotide dans un vecteur d'expression, et le procédé comprenant facultativement en outre la transformation de cellules avec les vecteurs d'expression.

3. Procédé selon la revendication 2, le procédé comprend en outre le criblage des cellules transformées pour l'activité des polypeptides codés par les variants de polynucléotide, et comprend facultativement en outre l'isolement d'au moins un polypeptide codé par les variants de polynucléotide.

4. Procédé selon l'une quelconque des revendications précédentes, dans lequel les différences de séquence d'acides aminés définies dans les polypeptides codés par les variants de polynucléotide sont des mutations identifiées comme étant associées à des changements d'une propriété souhaitée du polypeptide de référence.

5. Procédé selon l'une quelconque des revendications précédentes, le procédé étant utilisé :
(i) pour synthétiser des polynucléotides codant pour des variants d'enzyme ayant une propriété améliorée sur la base d'un ensemble de mutations connues pour affecter une propriété de l'enzyme, et facultativement pour synthétiser des polynucléotides codant pour des variants d'enzyme ayant des propriétés améliorées sur la base d'un ensemble de mutations connues pour affecter différentes propriétés de l'enzyme ; ou
(ii) pour synthétiser des polynucléotides codant pour des polypeptides ayant différentes permutations de combinaisons de mutations connues pour affecter différents aspects de la fonction d'un récepteur.

6. Procédé selon la revendication 1, dans lequel :
(i) la pluralité de variants de polynucléotide comprend au moins 10, au moins 25, au moins 35, au moins 50, au moins 75, au moins 90, au moins 120, au moins 150, ou au moins 180, variants de polynucléotide différents ;
(ii) chacun de la pluralité de variants de polynucléotide comprend au moins 2, au moins 3, au moins 6, au moins 9, au moins 12, au moins 15, au moins 18, au moins 21, au moins 24, au moins 27, ou au moins 30, différences de nucléotide définies par rapport à la séquence de polynucléotide de référence ;
(iii) au moins l'un de la pluralité de variants de polynucléotide comprend au moins 2, au moins 3, au moins 6, au moins 9, au moins 12, au moins 15, au moins 18, au moins 21, au moins 24, au moins 27, ou au moins 30, différences de nucléotide définies par rapport à la séquence de polynucléotide de référence ;
(iv) au moins l'un de la pluralité d'ensembles d'amplicons comprend au moins 3, au moins 5, au moins 7, ou au moins 10, amplicons différents ;
(v) au moins l'un de la pluralité d'ensembles d'amplicons comprend un polynucléotide de pontage ne comprenant aucune différence de nucléotide par rapport à la séquence de polynucléotide de référence ;
(vi) les longueurs des amorces sens et antisens sont de 20 à 50 nucléotides, de 20 à 40 nucléotides, ou de 25 à 35 nucléotides ;
(vii) au moins 65 %, au moins 75 %, au moins 85 %, ou au moins 95 % de la pluralité de variants de polynucléotide synthétisés comprennent la séquence de référence de pleine longueur avec les différences de nucléotide définies introduites par les amorces utilisées dans le procédé ;
(viii) la longueur de la séquence de polynucléotide de référence est d'au moins 500 pb, au moins 750 pb, au moins 1000 pb, au moins 1250 pb, ou au moins 1500 pb ;
(ix) la pluralité de paires d'amorces sens et antisens comprend de 6 à 200, de 6 à 150, de 6 à 100, de 6 à 50, de 6 à 40, de 6 à 30, de 6 à 25, de 6 à 20, ou de 6 à 15, oligonucléotides différents ; et/ou
(x) la pluralité de paires d'amorces sens et antisens comprend 400 ou moins, 300 ou moins, 200 ou moins, 100 ou moins, 50 ou moins, ou 25 ou moins oligonucléotides différents.

7. Procédé selon la revendication 1, dans lequel des amorces qui s'hybrident à des séquences de liaison d'amorce qui flanquent les variants de polynucléotide sont ajoutées pour amplifier les variants de polynucléotide dans des réactions d'amplification supplémentaires, et dans lequel, facultativement :
(i) les amorces flanquantes incorporent des séquences de reconnaissance pour des enzymes de restriction ; et/ou
(ii) les amorces flanquantes comprennent des séquences qui permettent l'expression *in vitro* directe au moyen d'un système couplé de transcription-traduction pour la synthèse du produit protéique sans nécessiter la transformation dans un organisme hôte.

8. Banque adressable de variants de polynucléotide comprenant une pluralité de variants de polynucléotide synthétisés selon le procédé de la revendication 1, ayant chacun au moins une différence de nucléotide définie par rapport à une séquence de polynucléotide de référence, la banque comprenant au moins 10 variants de polynucléotide, dans laquelle au moins un des variants de polynucléotide comprend au moins deux différences de nucléotide définies par rapport à la séquence de polynucléotide de référence, dans laquelle le polynucléotide de référence code pour un polypeptide de référence et chacun de la pluralité de variants de polynucléotide code pour un polypeptide ayant au moins une différence de séquence d'acides aminés définie par rapport au polypeptide de référence, et la banque comprenant un variant de polynucléotide codant pour un polypeptide présentant une amélioration d'une propriété souhaitée d'un polypeptide de référence codé par le polynucléotide de référence.

9. Banque adressable de variants de polynucléotide selon la revendication 8, la banque comprenant au moins 25, au moins 35, au moins 50, au moins 75, au moins 90, au moins 120, au moins 150, ou au moins 180, variants de polynucléotide différents.

10. Banque adressable d'amplicons, dans laquelle chaque membre de la banque d'amplicons comprend au moins une différence de nucléotide définie par rapport à une séquence de polynucléotide de référence et une région chevauchante adjacente capable de se lier à la région chevauchante adjacente d'au moins un autre amplicon dans la banque, la banque comprenant au moins 5 amplicons différents, la banque d'amplicons comprenant des membres pour assembler deux ou plus de deux variants de polynucléotide de pleine longueur différents comprenant des différences de nucléotide définies par rapport à la séquence de polynucléotide de référence, dans laquelle le polynucléotide de référence code pour un polypeptide de référence et chacun de la pluralité de variants de polynucléotide code pour un polypeptide ayant au moins une différence de séquence d'acides aminés définie par rapport au polypeptide de référence, et la banque d'amplicons comprenant au moins un ensemble d'amplicons capables de se lier pour former la pleine longueur de la séquence de polynucléotide de référence et codant pour un polypeptide ayant une amélioration d'une propriété souhaitée d'un polypeptide de référence codé par le polynucléotide de référence.

11. Banque adressable d'amplicons selon la revendication 10, la banque comprenant au moins 10, au moins 20, au moins 30, au moins 40, au moins 50, au moins 75, ou au moins 100, amplicons différents.

12. Banque adressable d'amplicons selon la revendication 10, dans laquelle la séquence de polynucléotide de référence code pour un polypeptide de référence et la pluralité d'amplicons comprend des membres suffisants pour assembler toutes les différences de nucléotide possibles codant pour une pluralité sélectionnée de différences de résidu d'acide aminé.

13. Banque adressable de clones, chaque clone étant capable de générer un polypeptide variant différent ayant au moins une différence de séquence d'acides aminés définie par rapport à un polypeptide de référence, généré par clonage de la banque adressable selon la revendication 8 dans un système d'expression.

14. Banque adressable de cellules transformées, chaque cellule étant capable de générer un polypeptide variant différent comportant au moins une différence de séquence d'acides aminés définie par rapport au polypeptide de référence, généré par transformation de la banque adressable de clones selon la revendication 13 dans des cellules.

15. Système robotique comprenant une banque adressable de variants de polynucléotide selon la revendication 8, une banque adressable d'amplicons selon la revendication 10 et/ou une banque adressable de cellules transformées selon la revendication 14.
